# EUROPEAN PATENT APPLICATION

(11) **EP 3 502 259 A1**
(43) Date of publication of application: **26.06.2019**
(21) Application number: 17208540.9
(22) Date of filing: 19.12.2017
(51) Int. Cl.: C12N 15/82

(54) **A COMBINATIONAL STRATEGY FOR REDUCING RANDOM INTEGRATION EVENTS WHEN TRANSFECTING PLANTS**

(71) Applicant: Universiteit Leiden, 2311 EZ Leiden (NL); Academisch Ziekenhuis Leiden h.o.d.n. LUMC, 2333 ZA Leiden (NL)
(72) Inventor: The designation of the inventor has not yet been filed
(74) Representative: HGF B.V.

(57) **Abstract**

The present disclosure provides methods for transfecting plants and for expressing RNA or polypeptide molecules in plants. In particular, plants having reduced expression and/or activity of a component of the classical NHEJ pathway and a component of the POLQ pathway are transfected in order to reduce random integration events. The disclosure further provides transfected plants and plant progeny produced by the methods disclosed herein.

## Description

### FIELD OF THE INVENTION

The present disclosure provides methods for transfecting plants and for expressing RNA or polypeptide molecules in plants. In particular, plants having reduced expression and/or activity of a component of the classical NHEJ pathway and a component of the POLQ pathway are transfected in order to reduce random integration events. The disclosure further provides transfected plants and plant progeny produced by the methods disclosed herein.

### BACKGROUND OF THE INVENTION

Genetic modification of plants by transfection allows alterations in traits such as increased yield, disease and pest resistance, increased vegetative biomass, herbicide tolerance, nutritional quality, drought and stress tolerance, as well horticultural qualities such as pigmentation and growth, and other agronomic characteristics for crop improvement. In addition, genetic modification of plants has been used as a system for the expression of recombinant proteins.

Transfection of plants with nucleic acid is now a common practice that may be carried out by a number of different methods known in the art. However, one disadvantage of the present transfection methods is the production of a relatively large number of random DNA integration events in the host genome. Random integration can have unpredictable and/or deleterious effects on the host organism. Furthermore, integration of DNA into the plant genome is not always desirable, particularly when genetically modified (GMO) plants arouse environmental and political issues. Thus, there remains a need for developing improved systems for expressing transgenes in plants.

### SUMMARY OF THE INVENTION

One aspect of the disclosure provides a method for reducing random integration of transfected nucleic acid molecules in a plant cell, said method comprising transfecting a plant cell with a nucleic acid molecule, wherein expression and/or activity in said plant cell is reduced for a component of the classical non-homologous end joining pathway (NHEJ) pathway and wherein expression and/or activity in said plant cell is reduced for a component of the POLQ pathway, as compared to a wild-type plant cell.

A further aspect of the disclosure provides a method for transfecting a plant cell with a nucleic acid molecule, the method comprising transfecting a plant cell with a nucleic acid molecule, wherein expression and/or activity for a component of the classical NHEJ pathway is reduced and expression and/or activity of a component of the POLQ pathway is reduced, in said plant cell as compared to a wild-type plant cell.

In preferred embodiments, the methods are used for gene targeting. Preferably, the methods are for producing a plant cell that carries a DNA sequence at a specific site in its genome via genetic recombination.

A further aspect of the disclosure provides a method for expressing an RNA molecule or a polypeptide in a plant cell, the method comprising transfecting a plant cell with a nucleic acid molecule encoding said RNA molecule or polypeptide, wherein expression and/or activity of a component of the classical NHEJ pathway is reduced and expression and/or activity of a component of the POLQ pathway is reduced, in said plant cell as compared to a wild-type plant cell.

A further aspect of the disclosure provides a method for producing a plant expressing an RNA molecule or a polypeptide, the method comprising transfecting a plant cell with a nucleic acid molecule encoding said RNA molecule or polypeptide, wherein expression and/or activity of a component of the classical NHEJ pathway is reduced and expression and/or activity of a component of the POLQ pathway is reduced, in said plant cell as compared to a wild-type plant cell, and generating the plant from said plant cell.

Preferably, the nucleic acid molecule is transfected into a plant cell having reduced expression and/or activity of a component of the classical NHEJ pathway and reduced expression and/or activity of a component of the POLQ pathway. Preferably, the component of the classical NHEJ pathway is Ku80, Ku70 and/or Lig4. More preferably, the component of the classical NHEJ pathway is Ku80 or Ku70. Preferably, the component of the POLQ pathway is POLQ and/or mre11. More preferably, the component of the POLQ pathway is POLQ.

In some embodiments, said nucleic acid molecule is transiently transfected into the plant cell. Preferably, the nucleic acid comprises a plant expression cassette. Preferably, the plant expression cassette comprises a nucleic acid sequence that encodes a polypeptide or an RNA molecule and regulatory sequences to drive expression. Preferably, the nucleic acid is a nuclease. Preferably, the nucleic acid is a component of a Crispr/Cas system.

In some embodiments, said nucleic acid molecule is integrated via site-specific genetic recombination or homologous recombination in the chromosome of the plant cell (or rather via site-specific genetic recombination or homologues recombination). Preferably, such stable integration results in the expression of a heterologous polypeptide or RNA molecule. In some embodiments, the integration is the result of homologues recombination. In some embodiments such stable integration disrupts or modifies an endogenous gene. In some embodiments, the integration is the result of site-specific recombination.

In some embodiments, said plant cell comprises an antisense oligonucleotide specific for a pre-mRNA encoded by a gene selected from a component of the classical NHEJ pathway and/or a component of the POLQ pathway, or a double-stranded RNAi molecule specific for mRNA encoded by a component of the classical NHEJ pathway and/or a component of the POLQ pathway. In some embodiments, the plant cell has a mutated gene for a component of the classical NHEJ pathway and/or for a component of the POLQ pathway. In some embodiments, the plant has a knock-out of any one of the said genes.

In some embodiments, the plant cell has one or more mutated alleles for a component of the classical NHEJ pathway and/or a component of the POLQ pathway. Preferably, the expression and/or activity of said genes is reduced by at least 70% as compared to the wild-type genes.

In some embodiments, said plant produced by the methods of the above, comprises said transfected nucleic acid molecule.

A further aspect of the disclosure provides for a plant or plant cell produced by the methods disclosed herein. Preferably, said plants and plant cells comprise the nucleic acid molecule stably integrated into the genome via genetic recombination.

A further aspect of the disclosure provides for a plant or plant cell wherein expression and/or activity of a component of the classical NHEJ pathway is reduced and expression and/or activity of a component of the POLQ pathway is reduced, in said plant or plant cell as compared to a wild-type plant cell. The disclosure further provides the use of said plant cells for transfecting a nucleic acid molecule.

A further aspect of the disclosure provides a method of producing a plant or plant cell as described above, comprising mutating one or more alleles selected from a component of the classical NHEJ pathway and/or a component of the POLQ pathway in the plant or plant cell or providing the plant or plant cell with an inhibitory nucleic acid molecule or binding molecule for a component of the classical NHEJ pathway and/or a component of the POLQ pathway.

A further aspect of the disclosure provides use of the plant or plant cell as described above or produced as described above for producing a plant expressing an RNA molecule or a polypeptide, wherein the nucleic acid molecule encoding said RNA molecule or polypeptide is not integrated into the plant cell chromosome.

A further aspect of the disclosure provide use of the plant or plant cell as described above or a plant or plant cell produced as described above for producing a plant expressing an RNA molecule or a polypeptide, wherein the nucleic acid molecule encoding said RNA molecule or polypeptide is integrated via site-specific genetic recombination or homologous recombination in the chromosome of the plant cell.

### BRIEF DESCRIPTION OF THE DRAWINGS

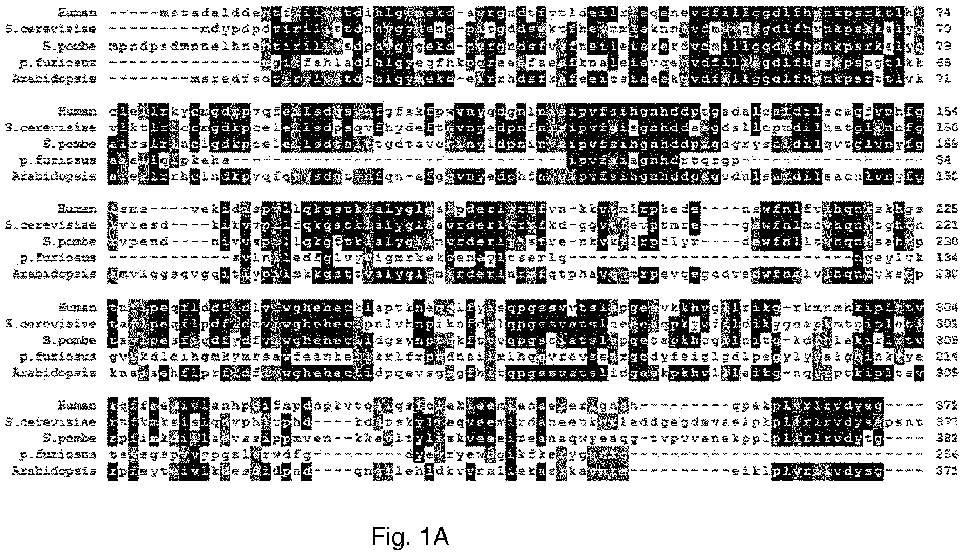

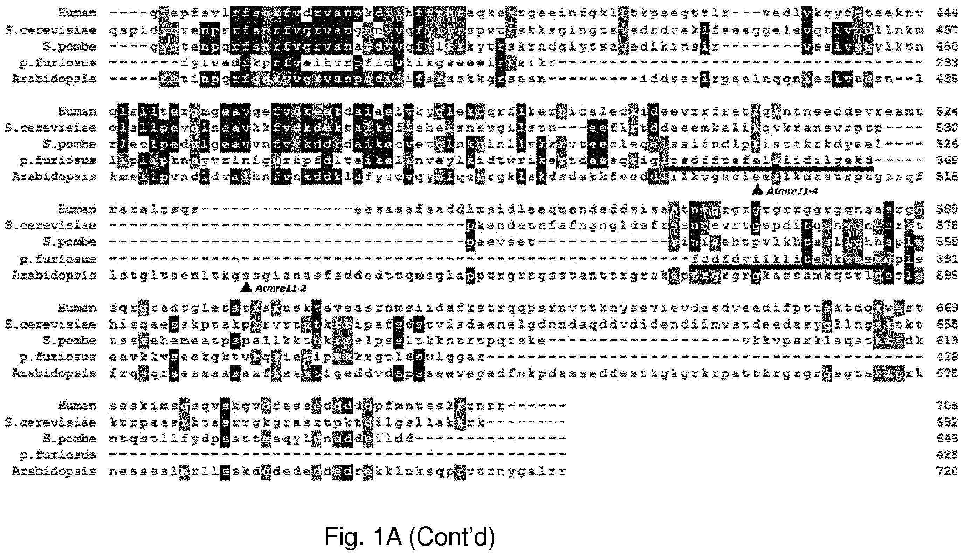

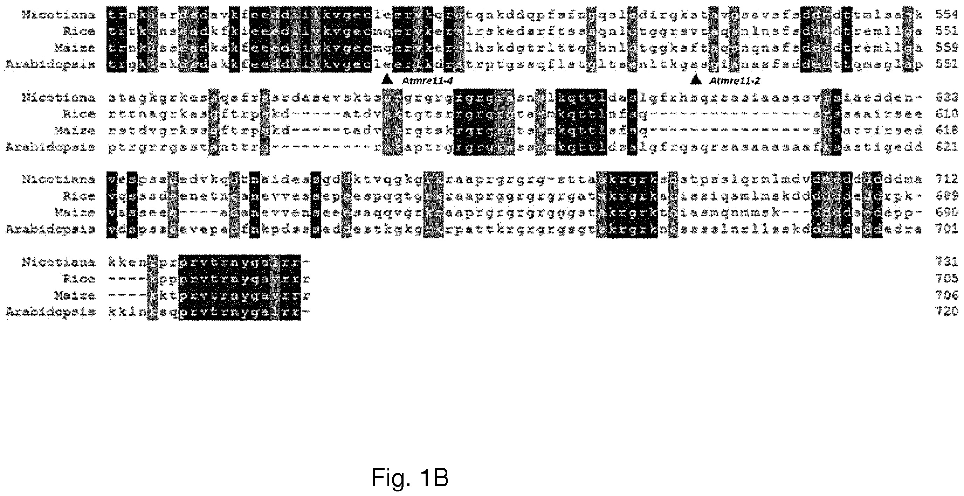

**Figure 1****. Alignment of Mre11. (A)** Alignment of Mre11 proteins from Humans, *Schizosaccharomyces pombe, S. cerevisiae* and *Arabidopsis.* (B) Alignment of Mre11 C-terminus from Nicotiana, Rice, Maize and Arabidopsis. The conserved amino acids are indicated with black boxes. The Rad50 binding domains (RBD) in *p. furiosus* are indicated with underlines.

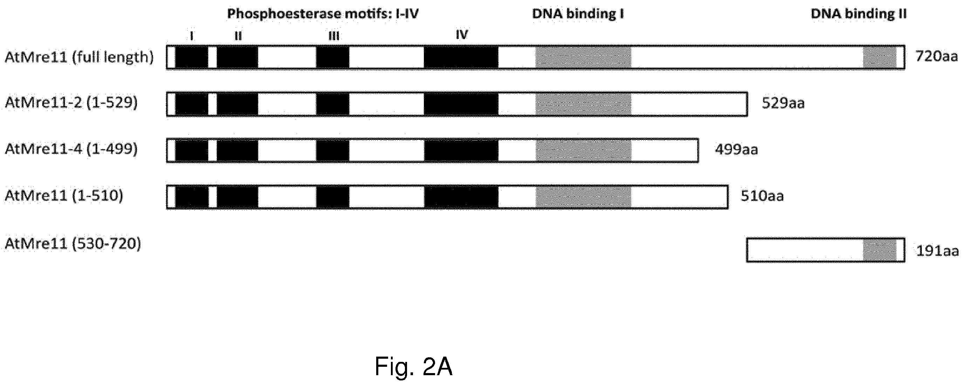

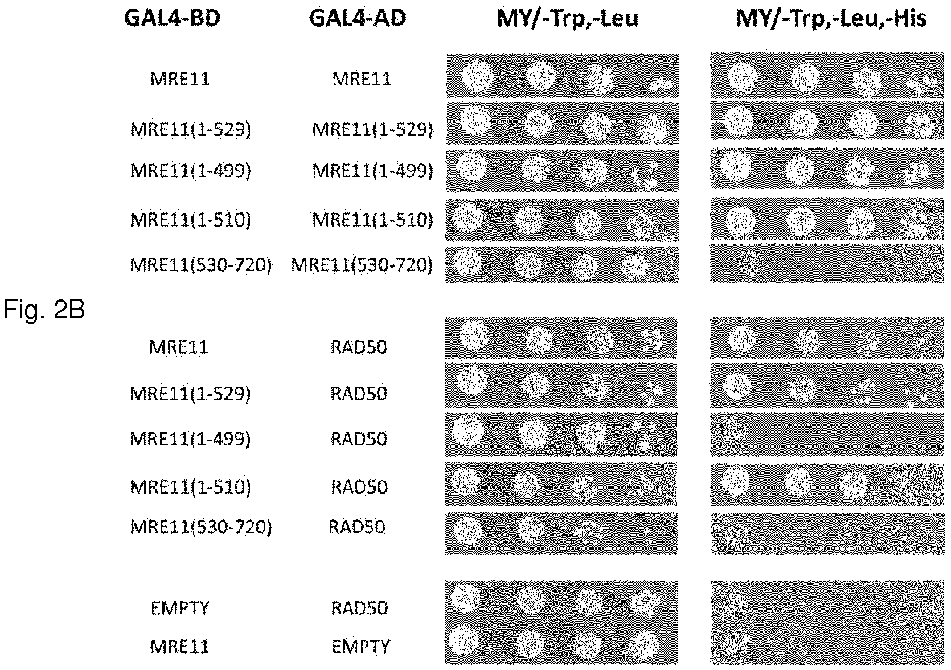

**Figure 2****. Yeast two-hybrid analysis of AtMRE11 interactions. (A)** Schematic regions of MRE11 analyzed in the *in vitro* interaction studies and the constructs used in the yeast two-hybrid analysis. **(B)** Interaction of MRE11 full length protein and deletions with RAD50 protein. The interactions between MRE11 and RAD50 or MRE11 itself resulting in histidine prototrophy.

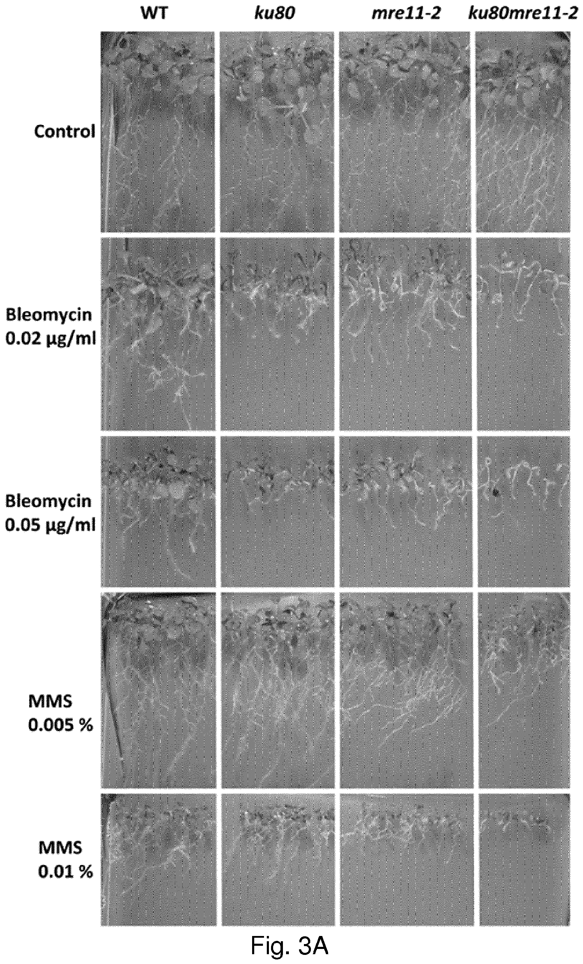

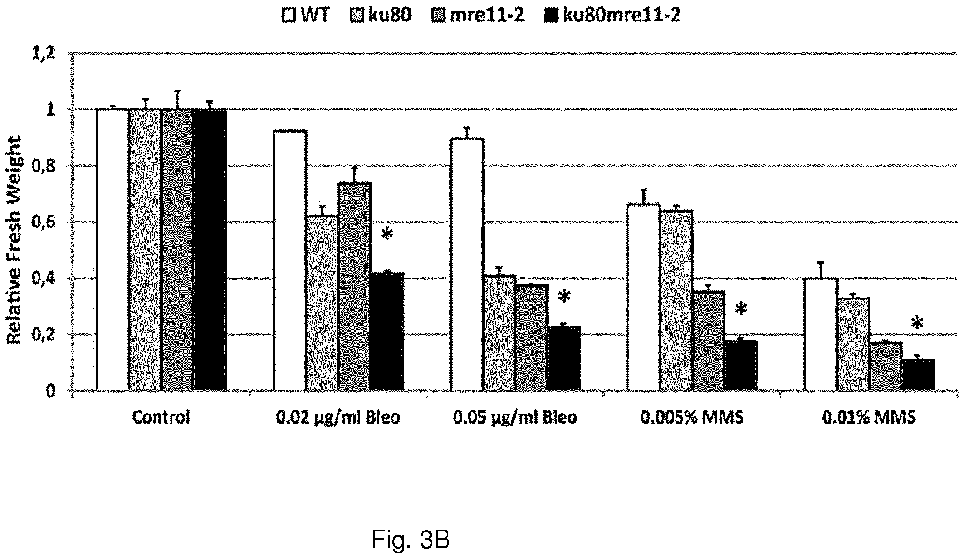

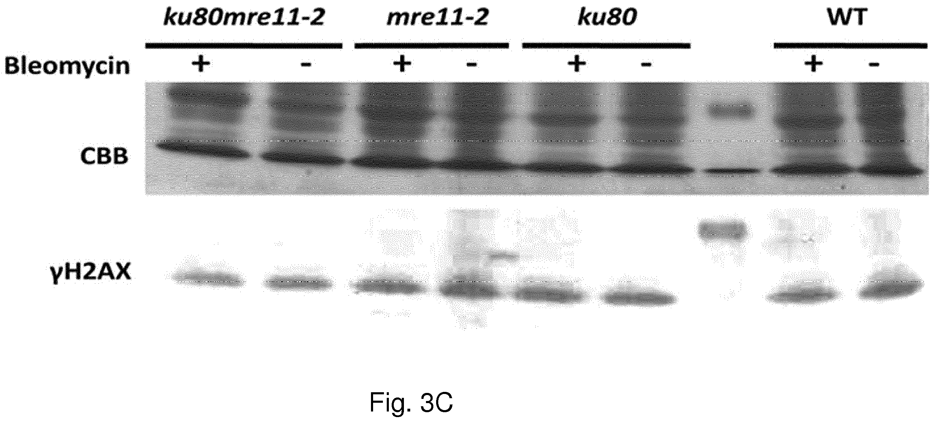

**Figure 3****. *Atku80mre11-2* double mutant hypersensitive to DNA damaging treatments. (A)** Phenotypes of wild-type plants and *Atku80, Atmre11-2* and *Atku80mre11-2* double mutants germinated on ½ MS medium (control) or ½ MS medium containing 0.02, 0.05µg/ml bleomycin or 0.005%, 0.01% MMS scored 2 weeks after germination. **(B)** Fresh weight of 10-d-old wild-type and *ku80, mre11-2* and *ku80mre11-2* double mutants treated with 0.02, 0.05 µg/ml bleomycin or 0.005%, 0.01% MMS. For each treatment 20 seedlings were weighted in duplicate. Fresh weight of the plants grown 10 days without bleomycin or MMS was set on 1. Error bar represent SD. Student's *t*-test; for *, *p*<0.01. (C) Western blot analysis of protein samples from the *ku80, mre11-2, ku80mre11-2* mutants and wild type (Col-0) plants using anti-γH2A antibody.

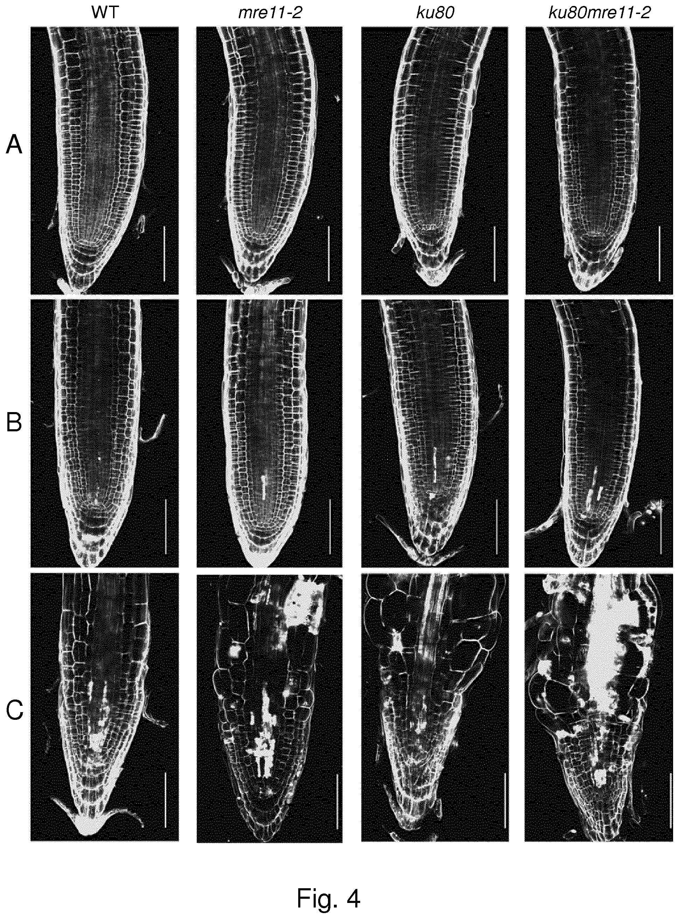

**Figure 4****. Cell death profile in root tips of mutants.** PI-staining root tips of 4-d-old wild-type (Col-0), *ku80, mre11-2* and *ku80mre11-2* seedlings grown on MS medium and transferred to MS medium with 0.05 µg/ml bleomycin for 1 day **(B)** and 4 days **(C),** and to MS medium without bleomycin for 2 days as control **(A).**

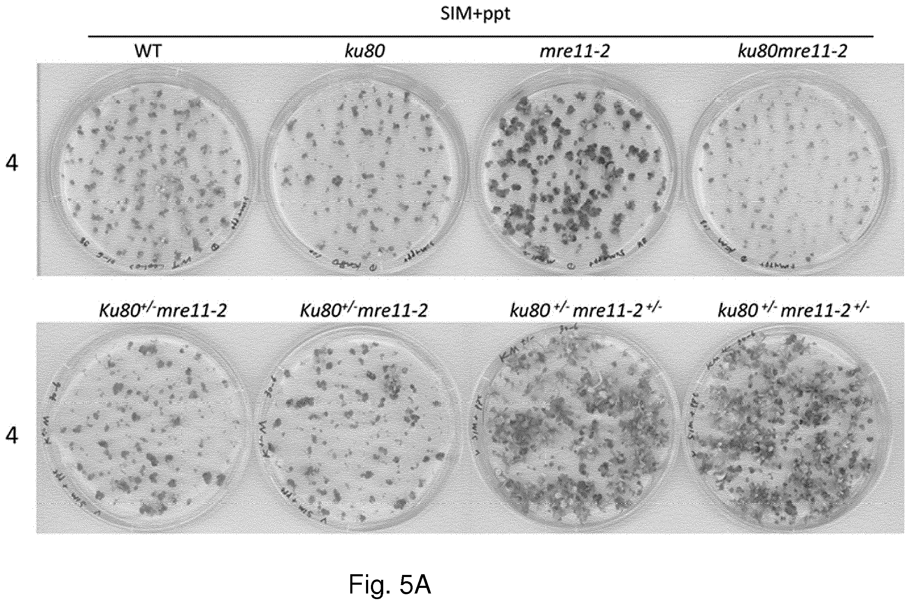

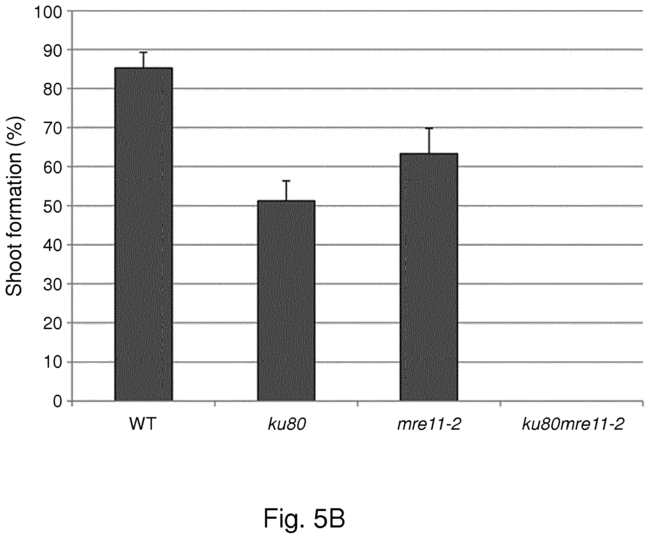

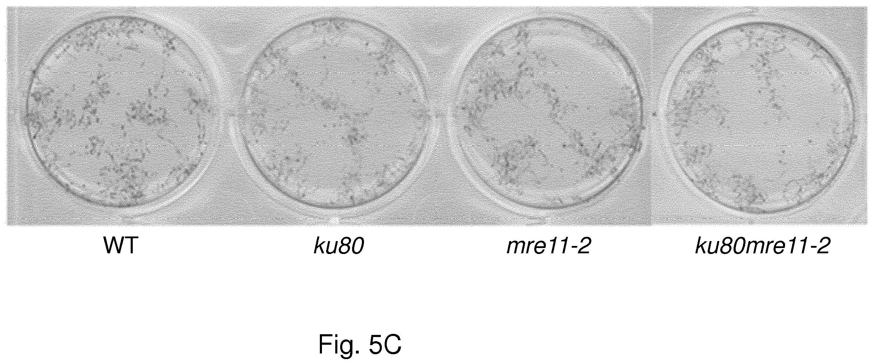

**Figure 5****. Root transformations of mutants.** (A) Root segments from wild-type and mutant plants were co-cultivated with Agrobacterium strain LBA1100 (pCambia3301) for 48 hours, and transferred to shoot induction medium with selection for phosphinothricin resistance. Photographs were taken 4 weeks after co-cultivation. (B) Shoot formation rate, represented by the percentage of green calli with shoot after 6 weeks cultivation on selection plates. (C) Root segments from wild-type and mutant plants were stained with X-Gluc overnight after 72 hours co-cultivation.

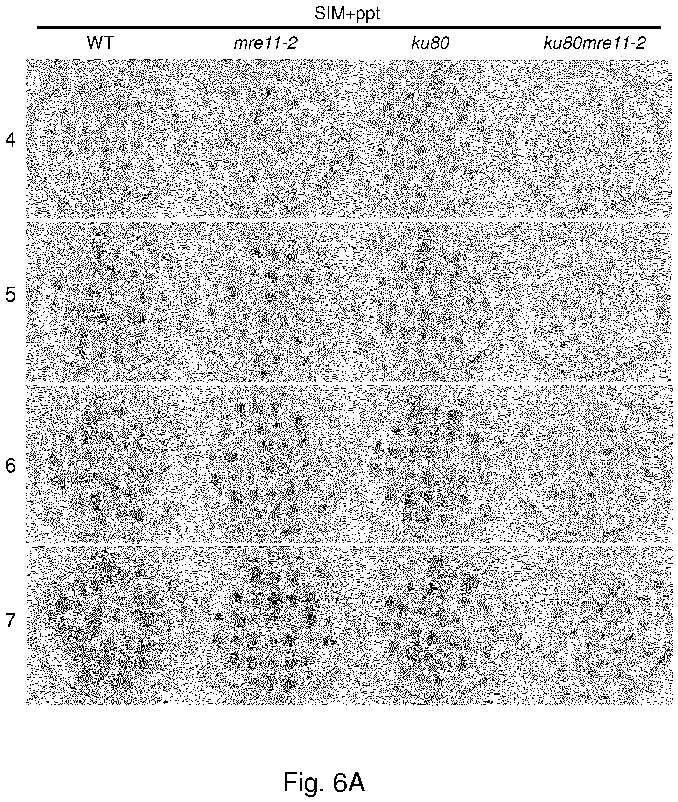

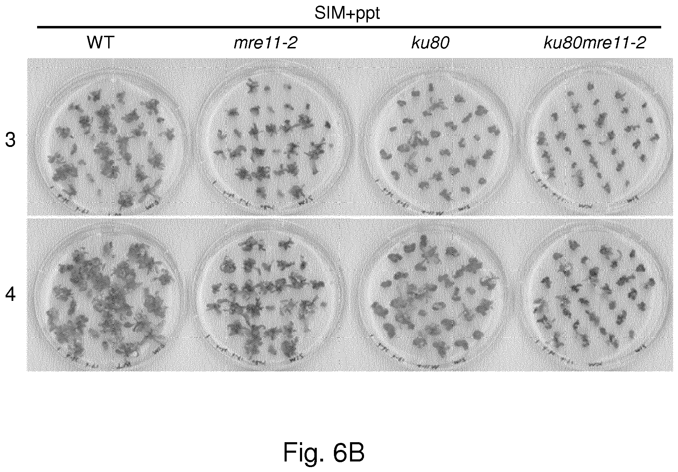

**Figure 6****. Shoot development on calli from root transformations.** (A) After 3 weeks growth on shoot induction medium with phosphinothricin selection, green calli were transferred to fresh shoot induction medium with phosphinothricin. (B) After co-cultivation with Agrobacterium, root segments were transferred to shoot induction medium without selection. After 3 weeks green calli were transferred to fresh shoot induction medium plates without selection. Photographs were taken every week after transfer to fresh plates. The number of 3, 4, 5, 6, 7 indicates the weeks of cultivation.

### DETAILED DESCRIPTION OF THE DISCLOSED EMBODIMENTS

In general, gene recombination in plants is mostly nonhomologous, or rather, introduced DNA is randomly inserted into any position of the chromosomes. Random integration can have deleterious effects if the introduced DNA disrupts, e.g., the expression of an endogenous gene. In addition, the expression of RNA or protein encoded by randomly integrated DNA is less predictable than by site-specific integration since both the site of integration and the number of integration events can affect expression. One of the objects of the methods disclosed herein is to reduce, avoid and/or eliminate random integration of transfected DNA in plants. An additional object of the methods is to provide an efficient method for gene targeting in plants.

In some instances, only transient transfection of DNA is desired in a plant. There may be, e.g., environmental or political reasons to avoid or limit the use of (stably) genetically modified plants. Additionally, in some situations, the desired expression may only be needed for a short time. One of the objects of the methods disclosed herein is to provide methods and plants in which the nucleic acid molecule of interest in only transiently transfected, or rather, the nucleic acid molecule of interest is not inserted into the plant chromosomes.

One aspect of the disclosure provides methods for transfecting a plant cell with a nucleic acid molecule of interest. The transfected plant cells can be used to express an RNA or a polypeptide of interest. Plants may be generated from such transfected plant cells. Methods are provided for reducing random integration of transfected nucleic acid molecules.

Plant cells have various methods to repair double stranded DNA breaks. These methods include non-homologous recombination and homologous recombination. Non-homologous recombination can lead to random integration. On the other hand, homologous recombination leads to guided repair, e.g., by the sister chromosome or by a transfected DNA molecule sharing homology with the region of the double strand DNA break. Non- homologous recombination can be facilitated by the classical non-homologous end joining pathway (NHEJ) or alternative NHEJ. The POLQ pathway functions as a part of the alternative NHEJ pathway in order to repair double stranded DNA breaks.

The methods disclosed herein comprise providing a nucleic acid molecule to a plant cell, wherein in the plant cell expression and/or activity of a component of the classical NHEJ pathway and a component of the POLQ pathway are reduced. As demonstrated in the examples, transfection of plants having reduced expression of a component of the classical NHEJ pathway, e.g. Ku80, and reduced expression of a component of the POLQ pathway, e.g. mre11, results in a significant reduction of random integration of the transfected nucleic acid molecule. This result is surprising since *ku80* mutant, or *ku70* mutant *Arabidopsis thaliana* plants and *mre11* mutant *Arabidopsis thaliana* plants show little to no reduction or even an increase in random integration of transfected nucleic acid molecules (Park et al., 2015).

The disclosure concerns methods and plants having reduced expression and/or activity of a component of the classical NHEJ (c-NHEJ) pathway and reduced expression and/or activity of a component of the POLQ pathway. Said components of the c-NHEJ pathway and the POLQ pathway are collectively referred to herein as "genes of interest". Preferably, the component of the classical NHEJ pathway is Ku80, Ku70 and/or Lig4. Preferably, the component of the POLQ pathway is POLQ and/or Mre11.

In some embodiments, the methods are preferred for transient expression of an RNA or polypeptide of interest. In other embodiments, the methods are preferred for the stable, site-specific integration of the nucleic acid molecule via genetic recombination. Transient expression refers to the expression of a nucleic acid molecule that is not integrated into the host chromosome, but functions independently. Stable integration refers to the integration of a nucleic acid into the host DNA by covalent bonds. "Genetic recombination" allows introduction in a genome of a selected nucleic acid at a defined site-specific position and includes both homologous recombination and site-specific recombination.

Preferably, the nucleic acid of interest is not inserted via non-homologous recombination. The methods disclosed herein are described as producing cells and plants in which the nucleic acid of interest is not integrated into any one of the plant cell chromosomes (i.e., transient transfection) or is integrated via site-specific genetic recombination or homologous recombination in any one of the chromosomes of the plant cell. It should be understood to the skilled person that when performing the methods disclosed herein a small percentage of plant cells may be produced in which the nucleic acid of interest has been inserted via non-homologous combination. However, a skilled person can easily identify and disregard such plant cells.

Any suitable nucleic acid molecule may be used for transfecting the plant cell. Although DNA is the preferred nucleic acid molecule, RNA transfection is also encompassed by the invention. For example, An et al. describes an RNA transfection method in Arabidopsis (An et al. Biosci Biotechnol Biochem. 2003 Dec;67(12):2674-7).

Preferably, the nucleic acid molecule comprises a nucleic acid sequence that encodes a polypeptide or an RNA molecule (e.g., a polypeptide encoding RNA or a non-coding RNA, e.g., long non-coding RNA, microRNA, tRNA, ribosomal RNA, snoRNA, etc.). Nucleic acid molecules of interest include those which impact plant insecticide resistance, disease resistance, herbicide resistance, nutrition and cellulose content, abiotic stress resistance, yield enhancement genes, drought tolerance genes, cold tolerance genes, antibiotic resistance, and marker genes. For example, US20140130207 describes RNAi molecules which can be expressed in plants in order to provide resistance to pests and pathogens.

Preferably, the transfected nucleic acid molecule is heterologous. As used herein, heterologous nucleic acid (or a heterologous gene) includes nucleic acid not normally found in the plant, for example nucleic acid from another species. Heterologous nucleic acid also includes a polynucleotide native to an organism that has been altered in some way (e.g., mutated, added in multiple copies, linked to a non-native promoter or enhancer sequence, etc.). Heterologous plant genes are distinguished from endogenous plant genes in that the heterologous gene sequences are typically joined to nucleotide sequences comprising regulatory elements such as promoters that are not found naturally associated with the gene for the protein encoded by the heterologous gene or with plant gene sequences in the chromosome, or are associated with portions of the chromosome not found in nature (e.g., genes expressed in loci where the gene is not normally expressed).

In some embodiments, the transfected nucleic acid molecule is a nuclease. Preferred nucleases include zinc-finger nucleases (ZFNs), transcription activator-like effector nucleases (TALENs), clustered regularly interspaced short palindromic repeat (CRISPR) nucleases, meganucleases, and nicking endonucleases. Such nucleases may be useful in methods of gene or genome editing. In preferred embodiments, the transfected nucleic acid is a nuclease or a component of the Crispr/Cas system.

Preferably, the transfected nucleic acid molecule is part of the Crispr/Cas system, such as a Cas nuclease and/or a guide nucleic acid. As is known to a skilled person, the Crispr/Cas system can be used for gene editing. Genes can be edited, mutated, replaced, or knocked-out using this system. Crispr/Cas can also be used to modulate gene expression by using modified "dead" Cas proteins fused to transcriptional activational domains (see, e.g., Khatodia et al. Frontiers in Plant Science 2016 7: article 506 and Ma et al. FEBS Journal 2014 5186-5193 for recent reviews of Crispr technology).

In preferred methods of the invention, a nucleic acid molecule encoding a Cas protein is transfected. The Cas protein may be a type I, type II, type III, type IV, type V, or type VI Cas protein. The Cas protein may comprise one or more domains. Non-limiting examples of domains include, a guide nucleic acid recognition and/or binding domain, nuclease domains (e.g., DNase or RNase domains, RuvC, HNH), DNA binding domain, RNA binding domain, helicase domains, protein-protein interaction domains, and dimerization domains. The guide nucleic acid recognition and/or binding domain may interact with a guide nucleic acid. In some embodiments, the nuclease domain may comprise one or more mutations resulting in a nickase or a "dead" enzyme (i.e., the nuclease domain lacks catalytic activity).

Preferred Cas proteins include c2c1, C2c2, c2c3, Cas1, Cas1B, Cas2, Cas3, Cas4, Cas5, Cas5e (CasD), Cash, Cas6e, Cas6f, Cas7, Cas8a, Cas8a1, Cas8a2, Cas8b, Cas8c, Cas9 (Csn1 or Csx12), Cas10, Cas10d, Cas10, Cas10d, CasF, CasG, CasH, Cpf1, Csy1, Csy2, Csy3, Cse1 (CasA), Cse2 (CasB), Cse3 (CasE), Cse4 (CasC), Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and Cul966, and homologs or modified versions thereof.

In preferred methods of the invention, a Crispr targeting sequence is transfected. Such sequences are known to the skilled person and include gRNA (guide RNA), crRNA, tracrRNA, and sgRNA. The Crispr targeting sequence binds to a complementary sequence in the host plant genome and targets a Cas protein to the respective site.

Transfection of the Crispr/Cas system in a plant cell having reduced expression and/or activity of a component of the classical NHEJ pathway in combination with reduced expression and/or activity of a component of the POLQ pathway, reduces the random insertion of Crispr/Cas components into the plant genome. While not wishing to be bound by theory, transient transfection of Crispr components may reduce off-targets effects and/or increase specificity of the Crispr/Cas system. In other embodiments, the disclosure provides methods for the generation of plants in which Crispr components (such as a Cas enzyme) are stably transfected via site-specific genetic recombination or homologous recombination.

In some embodiments, the methods provide transient transfection of the nucleic acid molecule. Preferably, the nucleic acid molecule for transient transfection comprises a plant expression cassette. A suitable plant expression cassette comprises 5'and 3' regulatory sequences operatively linked to a nucleic acid sequence encoding a transcript. The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter).

Preferably, a plant expression cassette comprises a promoter that drives expression in plants and a polyadenylation signal. Exemplary promoters for expression of a nucleic acid sequence include plant promoters such as the CaMV 35S promoter (Odell et al., 1985), CaMV 19S (Lawton et al., 1987), nos (Ebert et al., 1987), Adh (Walker et al., 1987), sucrose synthase (Yang and Russell, 1990), a-tubulin, actin (Wang et al., 1992), cab (Sullivan et al., 1989), PEPCase (Hudspeth and Grula, 1989) or those associated with the R gene complex (Chandler et al., 1989). Tissue specific promoters such as root cell promoters (Conkling et al., 1990) and tissue-specific enhancers (Fromm et al., 1986) may also be used. Examples of plant expression vectors include those detailed in: Becker, D. et al., 1992, New plant binary vectors with selectable markers located proximal to the left border, Plant Mol. Biol. 20: 1195-1197; and Bevan, M. W., 1984, Binary Agrobacterium vectors for plant transformation, Nucl. Acid. Res. 12:8711-8721; and Vectors for Gene Transfer in Higher Plants; in: Transgenic Plants, Vol. 1, Engineering and Utilization, eds.: Kung and R. Wu, Academic Press, 1993, S. 15-38.

In some embodiments, the methods provide stable site-specific integration of the nucleic acid molecule via genetic recombination. Site-specific integration refers to integration at a defined region of the genome that is dependent on the nucleic acid sequence in the genome. This differs from random integration. In one embodiment, the nucleic acid integrates into the plant genome via homologous recombination. Suitable methods and vectors for inducing homologous recombination are known. For example, a homologous recombination vector can be prepared comprising the nucleic acid molecule of interest flanked at its 5' and 3' ends by nucleic acid sequences which are homologous to endogenous plant sequences. Homologous recombination can be used, e.g., to replace a wild type gene on a chromosome by an unrelated new gene, an inactivated gene or a modified version of the wild-type gene (new allele). Homologous recombination may also be induced by nucleases such as zinc-finger nucleases (ZFNs), transcription activator-like effector nucleases (TALENs), clustered regularly interspaced short palindromic repeat (CRISPR) nucleases, meganucleases, and nicking endonucleases.

For example, the nucleic acid may encode a mutated form of an endogenous polypeptide or RNA molecule. The mutations may be gain of function or loss of function (e.g., inactivating mutation). In some embodiments, the nucleic acid may comprise, for example, a first region, a second region, and a third region. The first and third regions are substantially homologous to a gene of interest. The second region may comprise a mutated form of an endogenous gene or, e.g., encode a marker. Preferably, the marker is a positive selection marker, such as a drug resistance gene, a gene encoding a surface marker, a gene encoding a fluorescence marker, or a gene encoding β-galactosidase. Suitable homologous recombination methods and vectors for plants are known in the art and are described, e.g., in WO2003027261.

In one embodiment, the nucleic acid integrates into the plant genome via site-specific recombination. Several site-specific recombination systems have been tested in plants including the Cre/lox system, the Flp/FRT system, and the R/RS system. Recombinases exert their effects by promoting recombination between two of their recombining sites. In the case of cre, the recombining site is a Lox site, and in the case of Flp the recombining site is a Frt site. These recombining sites include inverted palindromes separated by an asymmetric sequence. Recombination between target sites arranged in parallel (so-called "direct repeats") on the same linear DNA molecule results in excision of the intervening DNA sequence as a circular molecule. Plants and plant cells carrying stable recognition sequences (e.g., FRT, lox, or RS site) in their genome can be used to transfect a nucleic acid molecule of interest flanked by the respective recognition sequence together with the appropriate recombinase (e.g., Flp, Cre, or R recombinase) in methods disclosed herein. Transfection results in the stable integration of the nucleic acid molecule.

For either transient or stable transfection, selectable or screenable markers can be included in the nucleic acid molecule to be transfected. "Marker genes" are genes that impart a distinct phenotype to cells expressing the marker protein and thus allow transfected cells to be distinguished from cells that do not have the marker. Such genes may encode either a selectable or screenable marker, depending on whether the marker confers a trait which one can "select" for by chemical means, i.e., through the use of a selective agent (e.g., a herbicide, antibiotic, or the like), or whether it is simply a trait that one can identify through observation or testing, i.e., by "screening" (e.g., the green fluorescent protein). Selectable marker genes include genes encoding antibiotic resistance, such as those encoding neomycin phosphotransferase II (NEO) and hygromycin phosphotransferase (HPT) as well as genes conferring resistance to herbicidal compounds. Herbicide resistance genes generally code for a modified target protein insensitive to the herbicide or for an enzyme that degrades or detoxifies the herbicide in the plant before it can act. (See, DeBlock, et al., (1987) EMBO J. 6:2513-2518; DeBlock, et al., (1989) Plant Physiol. 91:691-704; Fromm, et al., (1990) 8:833-839; Gordon-Kamm, et al., (1990) 2:603-618). For example, resistance to glyphosate or sulfonylurea herbicides has been obtained by using genes coding for the mutant target enzymes, 5-enolpyruvylshikimate-3-phosphate synthase (EPSPS) and acetolactate synthase (ALS). Many examples of suitable marker proteins are known to the art and can be employed in the practice of the invention. In preferred embodiments of the methods, the transfected plant cells or the regenerated plants thereof are screened for the presence of a selectable or screenable marker.

"Transfection" is defined herein as a process for introducing a nucleic acid into a plant cell and includes the term "transformation". Plant cells and plant parts include single cells and tissues from pollen, ovules, leaves, embryos, roots, root tips, anthers, flowers, fruits, stems shoots, and seeds; as well as pollen, ovules, leaves, embryos, roots, root tips, anthers, flowers, fruits, stems, shoots, scions, rootstocks, seeds, protoplasts, calli, and the like. Transfection may occur under natural or artificial conditions using various methods well known in the art. Suitable methods include viral infection, electroporation, lipofection, and particle bombardment. Examples of these techniques are described by Paszkowski et al., EMBO J. 3: 2717-2722 (1984), Potrykus et al., Mol. Gen. Genet. 199: 169-177 (1985), Reich et al., Biotechnology 4: 1001-1004 (1986), and Klein et al., Nature 327: 70-73 (1987). Transfection includes the introduction of nucleic acid into a plant cell by physical or chemical methods or through viral infection. However, it is clear to a skilled person that this process does not include the introduction of a nucleic acid by interbreeding or crossing.

The transfected cells may be regenerated to whole plants using standard techniques known in the art. In addition to transfecting in vitro cultivated plant cells, tissues or organs; whole living plants can also be transfected. Agrobacterium-mediated transfer is a widely applicable system for introducing genes into plant cells because the DNA can be introduced into whole plant tissues. Suitable processes include dipping of seedlings, leaves, roots, cotyledons, etc. in an Agrobacterium suspension which may be enhanced by vacuum-infiltration as well as for some plants the dipping of a flowering plant into an Agrobacteria solution (floral dip), followed by breeding of the transformed gametes.

The successfully transfected cells, which are preferably identified by selection or screening and cultured in an appropriate medium that supports regeneration, will then be allowed to regenerate into plants. "Regeneration" refers to the process of growing a plant from a plant cell (e.g., plant protoplast or explant) and such methods are well-known in the art.

In a preferred embodiment, the nucleic acid molecule of interest is transfected via Agrobacterium T-DNA system. Suitable Agrobacterium strains include LBA4404, EHA101, EHA105, AGL1, or GV3101. The T-DNA may be a modified Ti plasmid or an artificial vector derived from the Ti plasmid (tumour inducing plasmid), referred collectively herein as a "T-DNA vector". The Ti plasmid is a circular DNA molecule comprising a T-DNA region and a vir (virulence) region. The endogenous T-DNA region comprises the genes for the biosynthesis of auxin (aux), cytokinin (cyt) and opine (ocs) flanked by a right and left border. The borders are imperfect direct repeats having 24 base pairs The genes in the virulence region are responsible for transferring the T-DNA into plant cells. For example, the VirD2 endonuclease with assistance of VirD1 cuts at the borders of the T-DNA, releasing a single stranded copy of the T-DNA, the T-strand, which is transported into plant cells by the virB encoded transport system. The VirE2 protein binds the T-strand in the host cell and the complex enters the plant cell nucleus.

In some embodiments, the nucleic acid of interest is inserted into the T-DNA region of a Ti plasmid. Preferably, the aux, cyt, and ocs genes of the Ti plasmid are removed (i.e., the plasmid is "disarmed").

In some embodiments, an artificial vector derived from the Ti plasmid is used. The use of Agrobacterium-mediated plant integrating vectors to introduce DNA into plant cells is well known in the art. See, for example, the methods described by Fraley et al., (1985), Rogers et al., (1987) and U.S. Pat. No. 5,563,055, specifically incorporated herein by reference in its entirety. In some embodiments, one or more genes of the Ti plasmid is mutated to increase transformation efficiency, e.g., such as Agrobacterium strains wherein the vir gene expression and/or induction thereof is altered due to the presence of mutant or chimeric virA or virG genes (e.g. Chen and Winans, 1991, J. Bacteriol. 173: 1139-1144; and Scheeren-Groot et al., 1994, J. Bacteriol. 176: 6418-6246). In another embodiment, the Agrobacterium strain can comprise an extra virG gene copy, such as the super virG gene derived from pTiBo542.

In a preferred embodiment, a T-DNA binary system is used. In a binary system, the vir genes required for transfer of T-DNA into plant cells and the T-DNA are on separate plasmids. The "binary plasmid" comprises the nucleic acid of interest and, preferably a plant marker, flanked on the left and the right by T-DNA border sequences. The binary plasmid normally also comprises one ore more origins of replication to allow for replication in both E.coli and Agrobacterium and a bacterial selectable marker. The "helper plasmid" comprises the vir genes from the Ti plasmid. T-DNA binary system vectors are commercially available. A binary vector system wherein the T-region is located on the chromosome of the Agrobacterium strain has also been disclosed (see, e.g., EP-B 176 112).

In preferred embodiments of the methods disclosed herein, the methods comprise providing a plant cell with a nucleic acid molecule, said molecule comprising a nucleic acid sequence of interest flanked by a right border sequence (RB) derived from the Agrobacterium T-DNA sequence and a left border sequence (LB) derived from the Agrobacterium T-DNA sequence. As discussed herein, the nucleic acid molecule may further comprise regulatory sequences to drive expression in plants and/or marker genes. Preferably, a vir domain derived from the Ti plasmid is also provided in the methods. The vir domain may be present on the same nucleic acid molecule described above or may be provided by a separate vector, e.g., a helper plasmid. It is clear to a skilled person that the entire vir domain from the Ti plasmid is not necessary.

While not wishing to be bound by theory, we believe that the single-stranded unprotected T-DNA 3' end (left border) is normally a substrate for POLQ-mediated repair reaction, which leads to random integration. Reduction of the classical NHEJ pathway and the POLQ pathway in the plant cell reduces or eliminates the random integration of T-DNA, while leaving genetic recombination and transient expression unaffected. While not wishing to be bound by theory, the reduction of classical NHEJ and the POLQ pathway, is expected to reduce or eliminate random integration when a plant cell is transfected with nucleic acid which results in the presence in the nucleus of a single-stranded DNA with an unprotected 3' end.

In principle all plants can be used for transfection. Preferred transgenic plants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus cotton, cantaloupe, radicchio, papaya, plum, peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassaya, potato, sugarbeet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetable), buckwheat, Jerusalem artichoke, broad bean, vetches, lentil, dwarf bean, lupin, clover and Lucerne for mentioning only some of them. Preferably, the plant is Corn, Oilseed Rape, Canola, Cotton, Potato, Soybean , Sugar Beet, Squash, Cantaloupe, Rice, Flax, Raddicchio, Papaya, Alfalfa, or Wheat. Most preferred plants are Corn, Cotton, Soybean, Canola and Rice. In a preferred embodiment, the plant cell is not *Arabidopsis thaliana.*

Plants, like all multi-cellular eukaryotes, express polymerase theta, encoded by the POLQ (Pol θ) gene. In the present disclosure, POLQ and polymerase theta are used interchangeably. POLQ comprises a central domain having about 800 residues in plants and a polymerase domain which belongs to the "A" family of DNA polymerases (see, e.g., Yousefzadeh and Wood DNA Repair 2013 12:1-9). The polymerase domain comprises 5 "motifs" (see Figure 2A of Yousefzadeh and Wood). Sequence homology between plants is especially conserved in these regions, in particular in motifs 2, 5, and 6.

An exemplary POLQ sequence from plants is the "helicase and polymerase containing protein TEBICHI" from *Arabidopsis thaliana.* The 2154 amino acid protein sequence may be found on the NCBI database under accession number BAD93700.1. The TEBICHI sequence was also published in Inagaki et al. (Plant Cell 18 (4), 879-892 (2006)). The POLQ genes in other plants may be identified, e.g., by performing a BLAST alignment search with the POLQ sequence from *Arabidopsis thaliana.* For example, the POLQ genes from the following exemplary plants are listed in the NCBI database under the following accession numbers:
rice *Oryza sativa:* XP_015619406
potato *Solanum tuberosum:* XP_006356662
soybean *Glycine max:* XP_003545584
sugar beet *Beta vulgaris:* XP_010667709
tomato *Solanum lycopersum:* XP_010325163
banana *Musa acumanatea:* Sequence ID: ref|XM_009385225.1|
apple *Malus x domestica*: Sequence ID: ref |XM_008380001.1|
grape *Vitis vinifera:* Sequence ID: ref|XM_010650232.1|
rapeseed *Brassica napus:* Sequence ID: ref|XM_013882859.1|
Orange *Citrus x sanensas:* Sequence ID: ref|XM_006476088.2|
corn *Zea mays:* Sequence ID: AQK40086

While not wishing to be bound by theory, the disclosure provides that mre11 is also a member of the POLQ pathway. Plants express Mre11 encoded by the mre11 gene, like all multicellular eukaryotes. The Mre11 protein has been defined in the DNA damage response and combines with Rad50 and Nbs1 to form the MRN complex. Mre11 has both single-stranded DNA endonuclease activity and 3' to 5' exonuclease activity. Therefore, Mre11 comprises various domains including a DNA binding domain and nuclease domain which is also called N-terminal metalloprotease domain.

An exemplary mre11 sequence from plants is the "DNA repair and meiosis protein (Mre11)" from *Arabidopsis thaliana.* The 720 amino acid protein sequence may be found on the NCBI database under accession number CAB50793.1. The mre11 genes in other plants may be identified, e.g., by performing a BLAST alignment search with the mre11 sequence from *Arabidopsis thaliana.* For example, the mre11 genes from the following exemplary plants are listed in the NCBI database under the following accession numbers:
rice *Oryza sativa:* XP_015635669 or Gene ID: 4337137
potato *Solanum tuberosum:* XP_006341147 or Gene ID: 102587218
soybean *Glycine max:* XP_003539581 or Gene ID: 100801335
sugar beet *Beta vulgaris:* XP_010694433 or Gene ID: 104907235
tomato *Solanum lycopersum:* XP_004246548 or Gene ID: 101264663
banana *Musa acuminate:* XP_009412712 or Gene ID: 103994131
apple *Malus x domestics:* XP_008366397 or Gene ID: 103430037
grape *Vitis vinifera:* XP_010644252 or Gene ID: 100252167
rapeseed *Brassica napus:* XP_013730838 or Gene ID: 106434510
Orange *Citrus x sanensas:* XP_006464669 or Gene ID: 102624634
corn *Zea mays:* NP_001151499 or Gene ID: 100285133

Genes like Ku70, Ku80 and Lig4 are core components of the classical NHEJ pathway and are conserved in eukaryotes. Ku70 and Ku80 are shown to form a heterodimer which is involved in DNA repair and telomere maintenance. The Lig4 genes encode a DNA ligase protein, comprising a DNA ligase domain, that enables the pathway to ligate the DNA in the final step of the repair pathway. Further information regarding the classical NHEJ pathway in plants can be found in Saika et al. (2015, Frontiers in plant science, Volume 5).

The homologues of Ku70 and Ku80 were identified in Arabidopsis thaliana and published by Tamura et al. in Plant Journal (2002). These genes are described as ATP-dependent DNA helicase 2 subunit Ku70-like protein (KU70) and ATP-dependent DNA helicase 2 subunit Ku80-like protein (KU80). Together these 2 proteins can form a heterodimer. The 621 amino acid protein sequence of Ku70 can be found in the NCBI database under accession number BAD95294.1 and the 680 amino acid protein sequence of Ku80 can be found under accession number AAG44851.1. The Ku70 and Ku80 genes in other plants may be identified, e..g., by performing a BLAST alignment search with the sequence from *Arabidopsis thaliana.*
For example, the Ku70 genes from the following exemplary plants are listed in the NCBI database under the following accession numbers:
rice *Oryza sativa:* XP_015646738 or Gene ID: 4342584
potato *Solanum tuberosum:* XP_006364703 or Gene ID: 102580271
soybean *Glycine max:* XP_006606601 or Gene ID: 100775475
sugar beet *Beta vulgaris:* XP_010680644 or Gene ID: 104895758
tomato *Solanum lycopersum:* XP_004247997 or Gene ID: 101260670
banana *Musa acuminate:* XP_009397017 or Gene ID: 103981963
apple *Malus x domestic:* XP_008342867 or Gene ID: 103405626
grape *Vitis vinifera:* XP_010650420 or Gene ID: 100265198
rapeseed *Brassica napus:* XP_013660445 or Gene ID: 106365562
Orange *Citrus x sanensas:* XP_006477846 or Gene ID: 102627949
corn *Zea mays:* NP_001307810 or Gene ID: 100381505

For example, the Ku80 genes from the following exemplary plants are listed in the NCBI database under the following accession numbers:
rice *Oryza sativa:* XP_015631136 or Gene ID: 9267125,
potato *Solanum tuberosum:* XP_006339432 or Gene ID: 102579031
soybean *Glycine max:* XP_006580032 or Gene ID: 100781545
sugar beet *Beta vulgaris:* XP_010692265 or Gene ID: 104905416
tomato *Solanum lycopersum:* XP_004229815 or Gene ID: 101253129
banana *Musa acuminate:* XP_009399523 or Gene ID: 103983894
apple *Malus x domestica:* XP_008380667 or Gene ID: 103443574
grape *Vitis vinifera:* XP_010661633 or Gene ID: 100255780
rapeseed *Brassica napus:* XP_013725566 or Gene ID: 106429361
Orange *Citrus x sinensis:* XP_006490926 or Gene ID: 102626662
corn *Zea mays:* uncharacterized

An exemplary Lig4 sequence from plants is the "DNA ligase IV (AtLig4)" from *Arabidopsis thaliana.* The 1219 amino acid protein sequence may be found on the NCBI database under accession number AAF91284.1. The Lig4 genes in other plants may be identified, e.g., by performing a BLAST alignment search with the Lig4 sequence from *Arabidopsis thaliana.* For example, the Lig4 gene from *Oryza sativa* (rice) is listed in the NCBI database under the following accession number: XP_015636870 or Gene ID: 9269006

The BLAST family of programs which can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences.

Alternatively, standard molecular techniques may be used to identify the genes of interest disclosed herein from a particular plant species. For example, oligonucleotide probes based on the TEBICHI sequence can be used to identify the desired POLQ polynucleotide in a cDNA or genomic DNA library from a desired plant species. Probes may be used to hybridize with genomic DNA or cDNA sequences to isolate homologous genes in the plant species of interest.

Alternatively, the genes of interest disclosed herein can conveniently be amplified from nucleic acid samples using routine amplification techniques. For instance, PCR may be used to amplify the sequences of the genes directly from mRNA, from cDNA, from genomic libraries or cDNA libraries. PCR and other in vitro amplification methods may also be useful, for example, to clone nucleic acid sequences that code for proteins to be expressed, to make nucleic acids to use as probes for detecting the presence of the desired mRNA in samples, for nucleic acid sequencing, or for other purposes. Appropriate primers and probes for identifying the genes of interest disclosed herein in plants, can be generated based on the sequences of said genes. For a general overview of PCR see PCR Protocols: A Guide to Methods and Applications (Innis, M, Gelfand, D., Sninsky, J. and White, T., eds.), Academic Press, San Diego (1990).

In the plant cells used in the methods described herein, expression and/or activity is reduced for the genes of interest disclosed herein. The reduction in expression of said genes may occur at the level of nucleic acid or protein. Preferably, the amount of functional gene expression of said genes is reduced by at least 30%, 40%, 50%, 60%, 70%, 80%, 90%, 95% or 100% as compared to a corresponding wild-type plant cell. Preferably, the expression and/or activity is reduced by at least 50% as compared to a corresponding wild-type plant cell, more preferably, the expression and/or activity is reduced by at least 70%.

In some embodiments, expression of the genes of interest disclosed herein is determined by measuring the expression of said genes nucleic acid. Suitable methods include RT-PCR, quantitative PCR, Northern blotting, gene sequencing, in particular RNA sequencing, and gene expression profiling techniques, e.g., microarrays. In preferred embodiments, expression is determined by measuring the level of protein encoded by said genes. Suitable methods include ELISAs, immunocytochemistry, flow cytometry, Western blotting, proteomic, and mass spectrometry. Preferably, expression of the genes of interest disclosed herein, is determined in an immunoassay. Suitable immunoassays include, e.g., radio-immunoassay, ELISA (enzyme-linked immunosorbant assay), "sandwich" immunoassay, immunoradiometric assay, gel diffusion precipitation reaction, immunodiffusion assay, precipitation reaction, agglutination assay (e.g., gel agglutination assay, hemagglutination assay, etc.), complement fixation assay, immunofluorescence assay, protein A assay, and immunoelectrophoresis assay.

In some embodiments, POLQ activity refers to the ability of POLQ to bind DNA, in particular to chromatin. Such activity can be measured by any method known to a skilled person such as by immunoblotting chromatin fractions with a POLQ antibody as described in Fernandez-Vidal et al., 2014 Nature Communications 5.

In some embodiments, POLQ activity refers to its ability to act as a polymerase. Such activity can be measured, e.g., in the primer extension assay described in Hogg et al. Nucleic Acids Res. 2012 Mar; 40(6): 2611-2622, an MMEJ assay as described in Kent et al. Nat Struct Mol Biol. 2015 Mar; 22(3): 230-237. Zhan et al. (Nat Struct Mol Biol. 2015 Apr; 22(4): 304-311) describes additional assays to measure POLQ activity. As is clear to a skilled person, a reduction in POLQ activity refers to the reduction of activity as compared to the activity of wild-type POLQ from the same organism.

In some embodiments, Mre11 reduced activity may refer to the ability of Mre11 to bind DNA, in particular to chromatin, in particular to damaged DNA. Such activity can be measured by any method known to a skilled person such as by immunoblotting chromatin fractions with a Mre11 antibody. In some embodiments, mre11 reduced activity may refer to the nuclease activity of mre11.
In some embodiments, mre11 reduced activity may refer to disrupting the activity and/or expression of the C-terminal domain of mre11. In particular, the activity or expression of amino acids 530-720, with reference to AtMre11, is reduced or eliminated. In particular, the activity or expression of the glycine-arginine-rich GAR motif is reduced or eliminated. This motif is known to be arginine methylated and has been implicated in regulating DNA double-strand break processing (Yu et al. Cell Research 2012 22:305-320).In some embodiments, mre11 reduced activity may refer to reduced post-translational modification of mre11, preferably reduced methylation, preferably reduced methylation of the GAR motif.
In some embodiments, mre11 activity may refer to the ability to form a complex with Rad50 and Nbs1 in order to form the so called MRN complex.

As shown by Bundock and Hooykaas in 2002 (The Plant Cell, Volume 14) several motifs in mre11 are conserved among species. This is demonstrated with an alignment in Figure 1 including Arabidopsis, Humans, *Schizosaccharomyces pombe* (fungi) and *S. cerevisiae* (yeast). Preferred mutations may disrupt the C-terminus of the protein, preferably the glycine-arginine rich (GAR) motif (Dery et al., 2008, Yu et al., 2012). Preferred inhibitors inhibit the function of the C-terminus, preferably the GAR motif, of the Mre11 protein. In some embodiments, the inhibitors bind the C-terminus, preferably the GAR motif, of Mre11.

In some embodiments, Ku70 and/or Ku80 activity refers to the ability of Ku70 and Ku80 to form a heterodimer and/or to bind to damaged DNA. In a preferred embodiment, reduced activity of Ku70 or Ku80 prevents the proper functioning of the classical NHEJ pathway. As a result, the plant cell can no longer repair double stranded DNA breaks via this pathway.

In some embodiments, Lig4 activity refers to the ability of Lig4 to catalyse the ligation of the DNA during the process of classical NHEJ. In a preferred embodiment, reduced activity of Lig4 prevents the ligation of the DNA, thereby blocking the classical NHEJ pathway in the plant cell.

Down-regulation of the genes of interest disclosed herein may be accomplished by introducing a mutation that disrupts the gene by decreasing expression, by abrogating expression entirely, or by rendering the gene product non-functional of said genes. For example, the mutation may be a point mutation, an insertion, or a deletion, and the mutation may be located in a coding (e.g., in an POLQ exon) or non-coding portion of the said genes (e.g., in the POLQ promoter region).

Yousefzadeh and Wood (DNA Repair, Volume 12, Issue 10, 2013, Page 871) provide structural comparisons of POLQ family members and discuss the location of the catalytic domains. Preferred mutations disrupt the POLQ polymerase domain as depicted in Figure 2 of Yousefzadeh and Wood, which is hereby incorporated by reference. Preferably, the POLQ mutant reduces expression and/or activity by at least 50%, preferably at least by 70%, as compared to the wild-type gene.

Ku70 and Ku80 are evolutionary conserved among species as shown by the alignments in Tamura et al. (The Plant Journal, 2002). Furthermore, Jia et al. (Journal of Botany, Volume 2012) describes the characterization of two *Arabidopsis. thaliana* mutants in which AtKu70 or AtKu80 was inactivated through a T-DNA insertion. Preferred mutations may disrupt the heterodimerization of Ku70 and Ku80, the ability to bind to the DNA and/or the scaffolding function of the Ku proteins at the DNA ends. Preferably, the Ku70 and/or Ku80 mutant reduces expression and/or activity by at least 50%, preferably at least 70%, as compared to the wild-type gene.

Mutations in the genes of interest disclosed herein can be accomplished by any of the methods well known to those in the art including random mutagenesis methods such as irradiation, random DNA integration (e.g., via a transposon or T-DNA), or by using a chemical mutagen. Moreover, in certain aspects, a gene of interest may be mutated using a site-directed mutagenesis approach. In some embodiments, the said gene is mutated or disrupted using a homologous recombination vector or a targeted nuclease such as zinc-finger nucleases (ZFNs), transcription activator-like effector nucleases (TALENs), clustered regularly interspaced short palindromic repeat (CRISPR) nucleases, or meganucleases. Preferably. the CRISPR/Cas system is used. These methods are known in the art, and one of skill will be able to identify such methods as appropriate in light of the present disclosure. Several techniques are known to screen for specific mutant alleles, e.g., Deleteagene (Delete-a-gene; Li et al., 2001, Plant J 27: 235-242) uses polymerase chain reaction (PCR) assays to screen for deletion mutants generated by fast neutron mutagenesis, TILLING (targeted induced local lesions in genomes; McCallum et al., 2000, Nat Biotechnol 18:455-457) identifies EMS-induced point mutations, etc.

In a preferred embodiment, the plant cell has mutated genes of interest disclosed herein, preferably both alleles are mutated. In preferred embodiments, the mutation is a "knock-out" allele, i.e., no functional protein is produced.

In some embodiments, the CRISPR/Cas system is used to reduce expression and/or activity of the genes of interest disclosed herein. The CRISPR/Cas system is based on the RNA-guided Cas9 nuclease from the type II prokaryotic CRISPR (Clustered Regularly Interspaced Short palindromic Repeats) adaptive immune system (see, e.g., Belahj et al., Plant Methods 9:39, 2013). This system comprises three components: Cas9 protein, crRNA, and tracrRNA. The crRNA and tracrRNA are normally provided as a single RNA molecule referred to as gRNA (guideRNA). The guide RNA can be expressed using small nuclear RNA promoters such as U6 or U3. Plant codon-optimized versions of Cas9 have also been described and can be expressed in plants by either constitutive promoters (e.g. 35S promoter) or by a tissue specific or inducible promoter.

This system involves targeting Cas9 to the specific genomic locus via a gRNA. The canonical length of the gRNA is 20bp, however, for targeting plant loci, 19-22 bp may be used. The gRNA is designed to bind to the target sequence, in this case the genes of interest disclosed herein. The binding site is chosen such that the DNA targeted is followed by a PAM sequence (protospacer adjacent motif). The Cas9 protein from *Streptococcus pyogenes,* SpCas9, is often used in this system since it has a short PAM recognition sequence of NGG. Therefore, if SpCas9 is used, suitable gRNAs can be designed by screening the genomic locus of the genes of interest for the sequence (N)₁₉₋₂₂NGG. An online CRISPR Design Tool is also available to identify suitable target sites (http://tools.genome-engineering.org, Ren et al).

Further information regarding the use of the CRISPR/Cas9 system for inducing mutations in plants can be found in Lowder et al. Plant Physiology 2015 169:971-985; Belhaj et al. 2013 Plant Methods 9:39; Yin K, Gao C, Qiu JL, Progress and prospects in plant genome editing, Nature plants (2017); Liu X, Wu S, Xu J, Sui C, Wei J. Application of CRISPR/Cas9 in plant biology. Acta Pharm Sin B. (2017); and Arora L, Narula Gene Editing and Crop Improvement Using CRISPR-Cas9 System, A. Front Plant Sci. (2017)

The reduction in expression of the genes of interest disclosed herein can also be achieved using an "inhibitory nucleic acid molecule" whose presence in a cell causes the degradation of or inhibits the function, transcription, or translation of its target gene in a sequence-specific manner. Exemplary nucleic acid molecules include aptamers, siRNA, artificial microRNA, interfering RNA or RNAi, dsRNA, ribozymes, antisense oligonucleotides, and DNA expression cassettes encoding said nucleic acid molecules.

In some embodiments, the nucleic acid molecule is an antisense oligonucleotide. Antisense oligonucleotides (AONs) generally inhibit their target by binding target mRNA and sterically blocking expression by obstructing the ribosome. AONs can also inhibit their target by binding target mRNA thus forming a DNA-RNA hybrid that can be a substance for RNase H. AONs may also be produced as composite structures of two or more oligonucleotides, modified oligonucleotides, oligonucleosides, oligonucleotide mimetics, or regions or portions thereof. Such compounds have also been referred to in the art as hybrids or gapmers. Methods for designing and modifying such gapmers are described in, for example, U.S. Patent Publication Nos. 20110092572 and 20100234451. AONs typically comprise between 12 to 80, preferably between 15 to 40, nucleobases. Preferably, the AONs comprise a stretch of at least 8 nucleobases having 100% complementarity with the target mRNA.

In another method, a nucleic acid can be transcribed into a ribozyme or catalytic RNA, which affects expression of an mRNA. See, U.S. Pat. No. 6,423,885. Ribozymes can be designed to specifically pair with a target RNA and cleave the phosphodiester backbone at a specific location, thereby functionally inactivating the target RNA. Heterologous nucleic acids can encode ribozymes designed to cleave particular mRNA transcripts, thus preventing expression of a polypeptide. Hammerhead ribozymes cleave mRNAs at locations dictated by flanking regions that form complementary base pairs with the target mRNA.

Preferably, the nucleic acid molecule is a double-stranded RNAi molecule specific for mRNA encoded by the genes of interest disclosed herein. Preferably, the molecule comprises a fragment of the said genes to be silenced in an inverted repeat structure. The inverted repeats are separated by a spacer, often an intron. The RNAi construct is driven by a suitable promoter and transfected into a plant. Transcription of the transgene leads to an RNA molecule that folds back on itself to form a double-stranded hairpin RNA. This double-stranded RNA structure is recognized by the plant and cut into small RNAs (about 21 nucleotides long) called small interfering RNAs (siRNAs). siRNAs associate with a protein complex (RISC) which goes on to direct degradation of the mRNA for the target gene. A construct including a sequence that is operably linked to a regulatory region and a transcription termination sequence and that is transcribed into an RNA that can form a double stranded RNA, can be transformed into. Methods for using RNAi to inhibit the expression of a gene are known to those of skill in the art. See, e.g., U.S. Pat. Nos. 5,034,323; 6,326,527; 6,452,067; 6,573,099; 6,753,139; and 6,777,588. See also WO 97/01952; WO 98/53083; WO 99/32619; WO 98/36083; and U.S. Patent Publications 20030175965, 20030175783, 20040214330 and 20030180945. siRNAs directed to human POLQ have been described in Ceccaldi et al. Nature. 2015 Feb 12; 518(7538): 258-262.

Virus-induced gene silencing (VIGS) techniques are a variation of RNAi techniques that exploits the endogenous-antiviral defenses of plants. Infection of plants with recombinant VIGS viruses containing fragments of host DNA leads to post-transcriptional gene silencing for the target gene. In one embodiment, a tobacco rattle virus (TRV) based VIGS system can be used. Tobacco rattle virus based VIGS systems are described for example, in Baulcombe, Curr. Opin. Plant Biol. 2: 109-113 (1999); Lu, et al, Methods 30: 296-303 (2003); Ratcliff, et al, The Plant Journal 25: 237-245 (2001); and U.S. Pat. No. 7,229,829.

In some embodiments, activity of the genes of interest disclosed herein is reduced by providing the plant cell with a specific binding molecule. Preferably, gene activity is reduced by at least 30%, 40%, 50%, 60%, 70%, 80%, 90%, 95% or 100% as compared to a corresponding wild-type plant cell. Preferably, the binding molecule binds to the proteins of interest disclosed herein and inhibits its enzyme activity and/or its ability to bind DNA.

Preferably, the binding molecule is a small molecule, e.g. a chemical inhibitor. Additional binding agents include antibodies as well as non-immunoglobulin binding agents, such as phage display-derived peptide binders, and antibody mimics, e.g., affibodies, tetranectins (CTLDs), adnectins (monobodies), anticalins, DARPins (ankyrins), avimers, iMabs, microbodies, peptide aptamers, Kunitz domains, aptamers and affilins. The term "antibody" includes, for example, both naturally occurring and non-naturally occurring antibodies, polyclonal and monoclonal antibodies, chimeric antibodies and wholly synthetic antibodies and fragments thereof, such as, for example, the Fab', F(ab')2, Fv or Fab fragments, or other antigen recognizing immunoglobulin fragments.

Preferably, the binding molecule is a chemical inhibitor binding to any one of the proteins of interest disclosed herein. For example, mirin (B5389, Apex Bio) is a commercially available inhibitor for the Mre11-Rab50-Nbs1 (MRN)-ATM pathway.

Preferably, the binding molecule is an antibody or antigen binding fragment thereof binding to any one of the proteins of interest disclosed herein. Antibodies recognizing POLQ are commercially available (see, e.g., X3-Q588V7 [ABX] from Abmart which was generated using the TEBICHI protein). Antibodies recognizing Ku70 and/or Ku80 are described in the literature for example in Jia et al. (2012, Journal of Botany). Several antibodies recognizing Mre11 are commercially available, e.g., MRE11 Antibody 10744-1-AP (Protein Tech Rabbit polyclonal) and MRE-11 antibody (18) sc-135992 (Santa Cruz Biotechnology).

Antibodies which bind a particular epitope can be generated by methods known in the art. For example, polyclonal antibodies can be made by the conventional method of immunizing a mammal (e.g., rabbits, mice, rats, sheep, goats). Polyclonal antibodies are then contained in the sera of the immunized animals and can be isolated using standard procedures (e.g., affinity chromatography, immunoprecipitation, size exclusion chromatography, and ion exchange chromatography). Monoclonal antibodies can be made by the conventional method of immunization of a mammal, followed by isolation of plasma B cells producing the monoclonal antibodies of interest and fusion with a myeloma cell (see, e.g., Mishell, et al., 1980). Screening for recognition of the epitope can be performed using standard immunoassay methods including ELISA techniques, radioimmunoassays, immunofluorescence, immunohistochemistry, and Western blotting (Ausubel, et al., 1992) . In vitro methods of antibody selection, such as antibody phage display, may also be used to generate antibodies (see, e.g., Schirrmann et al. 2011). Preferably, a nuclear localization signal is added to the antibody in order to increase localization to the nucleus.

The present disclosure also encompasses a non-naturally occurring plant or plant cell wherein expression and/or activity of genes of interest disclosed herein, in said plant or plant cell is reduced as disclosed herein. In some embodiments, said plant or plant cell is not *Arabidopsis thaliana.* In a preferred embodiment, the plant or plant cell expresses a "inhibitory nucleic acid molecule" for any one of the genes of interest disclosed herein, as described herein. In a preferred embodiment, the plant or plant cell has a mutated gene of interest, as described herein. Said plants and plant cells are useful for carrying out the methods disclosed herein. Accordingly, the present disclosure also encompasses the use of the plant or plant cell for transfecting a nucleic acid molecule of interest.

The present disclosure also encompasses plant cells and plants produced by the methods disclosed herein. Said plants and plant cells are advantageous since they comprise the nucleic acid molecule, but their genome has limited or no random recombination events of the nucleic acid. In preferred embodiments, said plant cells and plants have reduced expression and/or activity of the genes of interest disclosed herein, as described herein.

The present disclosure also encompasses the progeny of plants produced by the methods disclosed herein. As used herein the term "progeny" denotes the offspring (including seeds) of any generation of a parent plant prepared in accordance with the methods described herein, wherein the progeny comprises the nucleic acid molecule of interest.

### Definitions

As used herein, "to comprise" and its conjugations is used in its non-limiting sense to mean that items following the word are included, but items not specifically mentioned are not excluded. In addition the verb "to consist" may be replaced by "to consist essentially of" meaning that a compound or adjunct compound as defined herein may comprise additional component(s) than the ones specifically identified, said additional component(s) not altering the unique characteristic of the invention.

The articles "a" and "an" are used herein to refer to one or to more than one (i.e., to at least one) of the grammatical object of the article. By way of example, "an element" means one element or more than one element.
All patent and literature references cited in the present specification are hereby incorporated by reference in their entirety.

As used herein, homologous recombination (HR) refers to error-free break repair using a non-damaged template (usually the sister chromatid).
As used herein, HDR (homology-driven repair) refers more broadly to the use of homologous sequences to direct repair using a template. A break can also be repaired if it is flanked by homologous sequences in which case the repair mode is called SSA, for single-strand annealing; this outcome is error prone.

All other repair that is not HR, HDR or SSA is usually referred to as end joining (EJ). Because EJ does not use homology to direct repair, it was originally termed non-homologous end-joining (NHEJ). The best known EJ pathway, which was first discovered, required a.o. the proteins KU70 and KU80 and LIG4 and is referred to as NHEJ or classical NHEJ (cNHEJ). EJ that is not cNHEJ (that manifested in cells that were deficient for cNHEJ) is termed "alternative" EJ or alt-EJ. Because this type of EJ frequently displays segments of identical bases at the junctions of the repair product, it has also been called: micro-homology-mediated EJ or MMEJ.

As used herein, TMEJ (pol Theta-Mediated EJ) refers to repair mediated by POLQ. While not wishing to be bound by theory, we propose that most alt-EJ repair is actually TMEJ mediated.

The invention is further explained in the following examples. These examples do not limit the scope of the invention, but merely serve to clarify the invention.

### EXAMPLES

### Example 1: Prevention of DNA integration in plants by the inactivation of a combination of plant factors

### Background

Genetic modification of plants is now routinely performed. Transformation can be done by various methods and vectors including Agrobacterium tumefaciens. It has been observed that transgenes integrate at fairly random positions via non-homologous recombination (NHR) in variable copy numbers in the plant genome. This may cause position effects (like silencing of transgenes) and mutation of genes at the integration site. Targeted DNA integration by homologous recombination (HR) would avoid such negative effects and would at the same time open the possibility to introduce by HR targeted mutations and edits into the genome. This is efficient in yeast, but a very rare event in somatic cells of higher plants. In fact integration by NHR is rare in yeast, strongly favoring integration by HR. A favored way to introduce genes into plant cells is through Agrobacterium-mediated transformation (AMT).

We have previously described methods for reducing random integration of transfected nucleic acid molecules by transfecting plant cells in which POLQ expression and/or activity is reduced (see WO 2017/164738 which is hereby incorporated by reference). Although the reduction of POLQ expression and/or activity greatly reduces the frequency of random integration, there may be situations where it is desired that such frequency be reduced even further.

In our previously reported experimental set-up using POLQ mutants, as described in WO 2017/164738, random integration is greatly reduced. A later study by Gelvin at al. confirmed a significant reduction of stable (random) transformation in POLQ mutant rice (Gelvin et al., Abstract book from 38th Crown Gall conference, Corvallis, USA; October 7-8 2017). A small amount of stable transformation in the mutant rice still occurred, at around 10-20% of the efficiency as observed in wild-type rice. One advantage of the embodiments disclosed herein is the further reduction of random transformation. In order to study pathways that would further decrease random integration, we choose to inhibit the POLQ pathway in a different manner. Mre11 mutants were used to inhibit the POLQ pathway.

The Mre11 protein has been well defined in DNA damage response (DDR) and homologous recombination (HR). It forms a complex with Rad50 and Nbs1 (Xrs2 in yeast) to form the MRN (MRX in yeast) complex and acts as a DSB sensor for activation of ATM and downstream signaling. Mre11 has both single-stranded DNA endonuclease activity and 3' to 5' exonuclease activity, and thus MRN plays a crucial role in promoting DSB end resection, which is likely to determine pathway choice between HR and NHEJ and repair outcome (see review Ceccaldi *et al.* 2016). However, the MRN/MRX complex, and Mre11 in particular, have been found to have also a direct role in NHEJ repair pathways. In plants, it has been shown that MRN mutants (Mre11, Rad50 and Nbs1) exhibit an increased sensitivity to DNA damage (Gallego *et al.* 2001; Bundock and Hooykaas 2002; Waterworth *et al.* 2007). Four *Arabidopsis thaliana* T-DNA insertion mutant lines of the *MRE11* gene were previously described: *mre11-1* and *mre11-2* (Bundock and Hooykaas 2002), *mre11-3* (Puizina *et al.* 2004), *mre11-4* ( 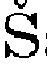 amanić *et al.* 2013). The *mre11-2* plants displayed normal growth and fertility, while *mre11-1, mre11-3* and *mre11-4* mutant lines had many developmental defects and were sterile. These reports demonstrated that Mre11 has essential roles in the response to irradiation-induced DSBs, meiotic recombination and maintenance of chromosomal stability. The Arabidopsis mre11-2 T-DNA insertion mutant has also been previously tested for transformation and gene targeting (GT) frequencies. The results showed a 5 fold lower transformation frequency and 3 GT events in about 3600 transformants whereas no GT events were found in about 11000 wild-type transformants (Jia et al. 2013). These experiments were performed with the floral dip transformation technique, which is more susceptible to reductions in transformation frequency than root transformation. While not wishing to be bound by theory, we suggest that Mre11 and POLQ act in the same or complementary pathways.

It has previously been shown that Ku70, Ku80, and Lig4, which are components of the classical NHEJ pathway and are conserved in all eukaryotes, are required for integration of the T-DNA by non-homologous recombination into the yeast genome (van Attikum et al. 2001). Genes involved in homologous recombination, like Rad51 and Rad52, were found to be required for the targeted integration of T-DNA by HR in the yeast genome (van Attikum et al. 2003). Inactivation of NHEJ in yeast prevented stable transformation unless the T-DNA showed homology to the yeast genome. In the absence of the NHEJ pathway, integration thus occurred exclusively by HR in yeast. In contrast to the results from yeast and fungi, NHEJ mutations do not appear to prevent T-DNA integration in plants. Although the transformation frequency using the floral dip method dropped significantly in such Arabidopsis mutants (Jia et al . 2013), less or no effect was seen in root transformation (Mestiri et al., 2014; Park et al., 2015). In contrast to the results in yeast, integration of the T-DNA still occurred efficiently by NHR in NHEJ mutant plants.

One possibility is that alternative (aNHEJ) or back-up (bNHEJ) end-joining pathways are functional in the NHEJ mutants of plants. In mammals and yeast such aNHEJ or bNHEJ pathways have been identified (Boulton and Jackson, 1996; Deriano and Roth, 2013), which seem to become more active in the absence of the core NHEJ factors, such as Ku and Lig4 (Nussenzweig and Nussenzweig 2007). Some of these pathways seem to employ microhomologies to anneal ends and therefore have also been called Microhomology-Mediated End Joining (MMEJ). Factors required for these alternative NHEJ pathways are among others PARP1/2, Lig3, and XRCC1 (Audebert et al. 2004). Orthologues for Parp1 and Xrcc1 have been identified in Arabidopsis and mutants could therefore be analyzed. Although involved in DNA repair, no significant change in transformation frequency was observed in these mutants, such as the parp1, parp2, ku70/80 triple mutant or the ku70/80 and xrcc1 double mutant (Charbonnel et al. 2010; Jia et al., 2013; Mestiri et al., 2014; Park et al., 2015). Our own experiments also revealed that the GT frequency is not significantly improved in all these Arabidopsis end-joining mutants.

We hypothesized that reducing activity of both the c-NHEJ pathway and the POLQ pathway would reduce random integration. To this end, we generated a homozygous double mutant *ku80mre11-2,* in which the c-NHEJ repair pathway is inactivated. More sensitivity to DNA damage stress was found in this double mutant revealing a crucial role for Mre11 in DNA repair in absence of Ku. Remarkably, the ku80mre11-2 double mutant was resistant to random DNA integration, while the single mutants still were proficient in random T-DNA integration. This suggests that Ku80 and Mre11 control different pathways of T-DNA integration, each of which functions independently in Agrobacterium T-DNA integration.

### Methods

### Plant material

The T-DNA insertional Arabidopsis thaliana mutants of ku80 and mre11-2, have been described previously (Bundock and Hooykaas 2002; Jia et al. 2012). The double ku80mre11-2 mutant was produced by crossing ku80 and mre11-2 homozygous mutants followed by PCR analysis of progeny for identification of double homozygous plants.

### Assays for sensitivity to bleomycin and methyl methane sulfonate (MMS)

Seeds of wild type, ku80, mre11-2 and ku80mre11-2 mutants were surface-sterilized as described (Weijers et al. 2001) and germinated on solid ½ MS medium without additions or containing 0.02 µg/ml or 0.05 µg/ml bleomycin (Sigma), 0.005% (v/v), 0.007% or 0.01% MMS (Sigma) and photographed after 2 weeks. After 10 days growth, the fresh weight (compared with controls) was determined by weighing the seedlings in batches of 20 in duplicate. Statistical analyses were performed using Prism version 5 (GraphPad Software Inc.).

### Cell Death Assay

Four days old seedlings on MS-agar plates were transferred to new MS-plates with DNA damaging agents. After one or four days treatments, the seedlings were placed in propidium iodide solution (PI, Sigma-Aldrich, 5 llg/ml in water) for 1 min and rinsed three times with water. Root tips were then transferred to slides in a drop of water and covered with a cover slip for observation under the fluorescence microscope.

### Western blottang

Seedlings of mutants grown in ½ MS for 2 weeks were transferred into MS medium with or without bleomycin for 4 hours. Then the seedlings were ground under liquid N2 in a Tissue-Lyser (Retch). One hundred µl protein extraction buffer (50 mM Tris-HCl pH 7.5; 2 mM EDTA; 0.2 mM PMSF; 1 mM DTT; 1×Protease inhibitor cocktail Complete®, EDTA free) was added to 50 mg of tissue powder. Soluble protein was isolated by centrifugation at 4°C. The protein concentration was determined using the BioRad protein assay reagent. Approximately 10 µg soluble proteins were mixed with sample buffer and boiled for 10 minutes, then loaded to a 10% SDS-PAGE gel for electrophoresis. The fractioned products were semi-dry blotted onto BA85 nitrocellulose membrane (Whatman). Equal loading of the gels and quality of protein preparations were checked by staining identical sets of gels with Coomassie Brilliant Blue R250. Blots were blocked for 3 hr in 5% nonfat dry milk in PBST (10 mM NaPO4, pH 7.4; 120mM NaCl; 2.7 mM KCl; 0.05% v/v Tween 20) and incubated overnight at 4°C with Rabbit anti γH2AX polyclonal antibody (1:1000) in the same buffer. The blots were washed 4 times with PBST and incubated for 3 h with anti-rabbit HRP antibodies (1:7500) (Promega). The blots were washed 4 times with PBST and detection 112 was performed using LumiGLO™ chemoluminescence detection kit (Cell Signalling).

### Yeast two-hybrid Assays

Full-length Mre11 was cloned into pACT2, while full length Rad50 was cloned into pAS2.1 (James et al. 1996). Mre11(1-499), Mre11(1-510), Mre11(1-529), Mre11(530-720) were amplified by PCR and cloned into pACT2 and pAS2.1. Primer sequences are presented in Table1. All PCR fragments were verified by sequencing. Interaction assays were performed by co-transformation of bait and prey plasmids into yeast strain PJ69-4A as previously described (Gietz et al., 1992), and plated on MY medium, supplemented with methionine, uracil, adenine and histidine and lacking leucine and tryptophan (MY/-LW). Subsequently, cells were incubated for 16 h in liquid MY/-LW and spotted onto selective solid MY medium lacking leucine, tryptophan and histidine (MY/-LWH) supplemented with increasing concentrations of 3-amino-1,2,4-triazole (3-AT, Sigma) ranging from 0 to 30 mM. Yeast cells were allowed to grow for 5 days at 30°C.

### Root transformation and transient GUS assay

Root transformation was performed as described (Vergunst et al. 2000). Root segments were infected with Agrobacterium LBA1100 (pCambia3301). After co-cultivation on callus induction medium containing 100 µM acetosyringone for 48 hours, root segments were washed, dried and incubated on shoot induction medium supplemented with 30 µg/ml phosphinothricin, 500 µg/ml carbenicillin and 100 µg/ml vancomycin. After 3-4 weeks, plates were photographed and transformation efficiencies were scored as infected root segments that produced any form of green callus. Statistical analyses 133 were performed using Prism version 5 (GraphPad Software Inc.). For transient GUS activity assays, root segments were washed after 72 hr co-cultivation, dried and stained with X-Gluc overnight at 37°C. Root segments were destained with 70% ethanol and visualized using a microscope.

### Results

### The C-terminal part of Mre11 contains the interaction domain with Rad50

The Arabidopsis Mre11 protein shared a large conserved domain in the N-terminus with Mre11 from other organisms, but there was less clear homology in the C-terminus (Figure 1A). Four Arabidopsis *MRE11* T-DNA insertion mutants have been characterized. The *mre11-2* mutant exhibited a normal growth phenotype and was fertile, whereas the *mre11-1, mre11-3* and *mre11-4* mutants were sterile and showed obvious morphological abnormalities. While the T-DNA insertions in *mre11-1* and *mre11-3* were localized in the N terminal part of the gene, the insertions in *mre11-2* and *mre11-4* were located in the C terminus. As a result of T-DNA insertion, 10 additional amino acids (NTQLKNVNMI) are added to the C-terminus of the predicted truncated protein in the *mre11-2* mutant, while 35 additional amino acids (ARRYRFSCLITFFNSGLLFQTGTTLNPFSGYSFDL) are predicted to be added to the truncated protein at the C-terminus in the *mre11-4* mutant. Except for the extra amino acids formed at the C-terminus, putative truncated Mre11 proteins formed in the *mre11-2* and *mre11-4* mutants would differ only by 30 amino acids. *In silico* analysis of the predicted truncated Mre11 proteins showed that the *mre11-4* deletion removes a large region that is highly conserved in Mre11 proteins of different plants, while only a small part of this is deleted in the Mre11 protein encoded by the *mre11-2* allele (Figure 1B). To get a better understanding of the functional differences between the Mre11-2 protein truncated at amino acid 529 and the Mre11-4 protein truncated at amino acid 499, yeast two hybrid assays were performed (Figure 2). Full-length Mre11 displayed a strong interaction with Rad50 and with itself, indicating that the Arabidopsis Mre11 can potentially homodimerize and form a Mre11-Rad50 complex as expected. To test if the truncated proteins formed in *mre11* mutants still could interact with Rad50 and homodimerize, each of four fragments of Mre11 (amino acids 1 to 529; 1 to 510; 1 to 499; 530 to 720) cloned in pAS2-1 and pACT2 were cotransformed into yeast. A full length clone of Rad50 in pACT2 and each of four fragments of Mre11 cloned in pAS2-1 were cotransformed into yeast. We found that amino acids 1 to 499 of Mre11 were sufficient for self-interaction. Although Mre11 amino acids 1 to 529 displayed a strong interaction with Rad50 comparable to that observed for full-length Mre11, the smaller version (amino acids 1 to 499) did no longer interact with Rad50. Apparently, presence of the area from amino acids 499 to 529 overlapping with a region that is strongly conserved in plant Mre11 is essential for the interaction of Mre11 with Rad50. Indeed, an Mre11 construct (amino acids 1 to 510) preserving this conserved region, but only 11 amino acids larger than the Mre11-4 protein Mre11 (amino acids 1 to 499) was now able to interact with Rad50. The C-terminal part of Mre11 (aa 530 to 720) by itself did neither homodimerize nor interact with Rad50. These results revealed that the ability to form Mre11 homodimers and Mre11-Rad50 complexes are preserved in the Mre11-2 protein, but that the Mre11-4 protein could no longer form a complex with Rad50. As Mre11 relies for most of its functions on the formation of a complex with Rad50 (and Nbs1), this explains the different phenotypes of the *mre11-2* mutant in comparison with *mre11-4* and the other T-DNA insertion mutants.

### The Ku80mre11-2 double mutant is more sensitive to DNA damage stress than each of single mutants

Previously, in our lab it was shown that the *ku80* and the *mre11-2* single mutants were hypersensitive to DNA damage stress (Bundock and Hooykaas 2002; Jia *et al.* 2013). In order to test whether the mre11-2 mutation affects the same or a different repair pathway as Ku80, the *ku80mre11-2* double mutant was obtained by crossing the single mutants and tested for sensitivity to genotoxic agents (Figure 3A). Without any treatments, the double mutant grew similarly as the wild type. However, upon treatment with bleomycin or MMS, the double mutant grew more slowly and developed fewer true leaves in the assay period than each of the single mutants, indicating increased DNA damage sensitivity in double mutant plants. We also evaluated the effect of DNA damage on the growth of 10-days-old plate-grown plants by quantification of the fresh weight (Figure 3B). We found that the fresh weight of double mutant plants was dramatically reduced in comparison with that of wild type and each of the single mutants, when treated with bleomycin or MMS.

As the *ku80mre11-2* double mutant plants might have difficulty in repairing DNA damage, this might lead to more cell death than in the wild type or each of the single mutants. To test this, we used propidium iodide (PI) staining to reveal cell death. As can be seen in Figure 4, no cell death was observed in wild type and mutants under the normal growth conditions.

Bleomycin treatment caused cell death in the root meristem of both wild type and the mutants, and this increased over time. More cell death was seen in the roots of the double mutant than in that of wild type and each of the single mutants (Figure 4B). When the time period of the genotoxic stress was extended, a much shorter meristematic zone was observed in the root tip of the double mutant (Figure 4C), which might be due to the enhanced cell death in the root meristem. Meanwhile, the epidermal cells of the double mutant were also more deformed and enlarged than those of wild type and each of the single mutants (Figure 4C).

Taken together, the results showed that *ku80mre11* double mutant plants grow similarly as wild type plants under normal growth conditions, but they are more sensitive to DNA damage and grow slower in the presence of genotoxic agents than each of the single mutants. This indicates that Mre11 probably plays an important role in Ku-independent DNA repair pathways in somatic plant cells.

### The Ku80mre11-2 double mutant is resistant to Agrobacterium mediated T-DNA integration

Previously, we found that both the *ku80* and *mre11-2* mutants were somewhat recalcitrant to transformation in the floral dip procedure (Jia *et al.* 2012), but other groups have shown that the *ku80* mutant is either not or little affected in floral dip or root transformation by *Agrobacterium* (Gallego *et al.* 2003; Park *et al.* 2015). In order to test whether the *ku80mre11-2* double mutant is affected more strongly in *Agrobacterium*-mediated T-DNA transformation, we used the *ku80mre11-2* double mutant together with wild type and single mutants in the root transformation procedure (Figure 5). To this end, roots were cocultivated with an *Agrobacterium* strain containing the pCambia3301 binary vector, and callus formation was selected on medium with phosphinothricin. The transformation frequencies of the single mutants were not significantly different from the wild type. The calli formed on the roots of the *mre11-2* mutant grew somewhat greener than those formed by the wild type. This was due to a difference in ecotype between the *mre11-2* mutant (ecotype Ws) and the *ku80* mutant (ecotype Col), but did not influence the transformation results. While wild type and single mutants were transformed at a similar frequency, surprisingly, the *ku80mre11-2* double mutant only produced very few green calli in the transformation procedure (Figure 5A). Upon longer incubation of these calli on selection plates no green shoots were formed and the calli eventually died (Figure 5B, 6A), indicating that the *ku80mre11-2* double mutant cannot be transformed by root transformation. One allele of Ku80 was sufficient for rescue as can be seen by the efficient transformation of roots with the ku80+/-mre11-2-/- genotype (Figure 5A). Also, calli and green shoots were formed normally on medium without selection, although calli of the double mutant grew a bit slower than those of wild type and single mutants (Figure 6B).

In order to test whether the lack of callus formation was due to the lack of T-DNA transfer or a deficiency in T-DNA integration, roots were transformed again by an Agrobacterium strain with the pCambia3301 binary vector, which contains the *gus* gene besides the *bar* gene providing resistance to phosphinothricin. Plant cells receiving the T-DNA from this vector express the *gus* and *bar* genes already before integration, which disappears in due time when the T-DNA is not stably integrated. Expression of the gus gene can be visualized , for instance by addition of a compound called X-Gluc to the growth medium. This colorless compound is converted in a blue pigment by the action of the GUS enzyme. The addition of X-Gluc to roots of the wild type, single mutants and the ku80mre11-2 double mutant showed blue staining after cocultivation in all cases, indicative of the expression of a transferred *gus* gene. These results thus demonstrate that T-DNA transfer is not affected in these mutants resulting in transient expression of the *gus* gene (Figure 5C), but indicate that the lack of stable transformation is due to a deficiency in T-DNA integration.

This suggests that Ku80 and Mre11 are involved in two different pathways of T-DNA integration, each of which is not essential, but after inactivation of both transformation is reduced to below detection level.

### Discussion

The predicted protein sequence of the Arabidopsis Mre11 orthologue contains a highly conserved N-terminal nuclease domain which is essential for repair in yeast and human, but a less conserved region in the Mre11 C-terminus. Three different T-DNA insertion lines of Arabidopsis *MRE11* have many developmental defects and are sterile suggesting that the functions of Arabidopsis Mre11 are severely compromised in these mutants. The fourth, mutant *mre11-2,* however, is fertile and grows normally. The T-DNA insertions in the *mre11-4* and *mre11-2* mutants both leave the N-terminal nuclease domain of the encoded Mre11 protein intact. Our results from two-hybrid analysis demonstrated that the interaction domain necessary for the formation of a Mre11-Rad50 complex is still present in the Mre11-2 protein, but absent in Mre11-4. Mre11 relies for most of its functions on the formation of a Mre11-Rad50-Nbs1 complex, explaining the phenotypic differences between the two mutants (Stracker and Petrini 2011). Rad50 binding domains (RBD) have been identified in the C-terminal part of *P.furiosus* Mre11 (Williams *et al.* 2011). Although this C-terminal region is less conserved, the C-terminal region of Mre11 in yeast and humans also have been indicated to be involved in the interaction with Rad50 (Chamankhah and Xiao 1999; Park *et al.* 2011). Our studies also point out that a region in the Arabidopsis Mre11 C-terminus likewise participates in Rad50 binding. The *mre11-2* mutant preserves the Rad50 binding activities, grows normally but still shows hypersensitivity to DNA damaging agents. This suggests that the deletion has a specific role in Arabidopsis DNA damage signaling and/or DNA repair.
A *ku80mre11-2* double mutant was generated by crossing the single mutants. The heterozygote ku80+/-mre11-2-/- and ku80+/-mre11-2+/- progeny was used as controls and behaved as expected. We found that the *ku80mre11-2* double mutant was more sensitive to DNA damage than each of the single mutants. The subsequent observation of more cell death events in this double mutant, when longer exposed to damage-inducing agents indicates that NHEJ repair pathways are severely disturbed in the concomitant absence of Ku80 and a fully functional Mre11. Beyond its function in activation of the DNA damage response, Mre11 has also been implicated in b-NHEJ that occurs in the absence of c-NHEJ (Zha *et al.* 2009). Our results corroborate that Mre11 plays an important role in the b-NHEJ repair pathways in plant cells and that the C-terminal part of Mre11 is important for this function .

Other evidence supporting a role of Mre11 in b-NHEJ was that the *ku80mre11-2* and also the *ku70mre11-2 (not shown)* double mutant was untransformable by *Agrobacterium.* Using yeast as a model it was shown in our lab that T-DNA integration in yeast (*Saccharomyces cerevisiae*) is dependent on the classical non-homologous end joining (c-NHEJ) pathway of DSB repair, and that proteins such as Ku70, Ku80 and DNA ligase IV are essential for T-DNA integration (van Attikum *et al.* 2001). Inactivation of c-NHEJ in yeast prevented random T-DNA integration, although there is a b-NHEJ pathway in yeast. Putative T-DNA integration by this b-NHEJ pathway may have been beyond the limits of detection. In subsequent studies in plants, T-DNA integration was invariably seen in all of the NHEJ mutants studied and even in double and triple mutants, such as *ku parp1, ku parp1 parp2, ku xrcc1* (Jia et al.,2013; Mestiri et al., 2014; Park et al., 2015), demonstrating that other important factors must be involved in T-DNA integration. The Mre11 protein might represent such an important protein that plays a critical role in in T-DNA integration as inactivating it together with Ku unexpectedly completely prevented T-DNA integration in plants. Therefore, Ku80 and Mre11 apparently control two different pathways, each of which is not essential, but together are responsible for all T-DNA integration in plants. A recent study showed that in Arabidopsis the Pol θ ortholog Tebichi (Teb) is essential for T-DNA integration (van Kregten et al., 2016). The Pol θ protein is known to mediate alternative end-joining via a pathway called Theta-mediated end-joining (TMEJ). Our results show that besides TMEJ, also c-NHEJ plays a role in T-DNA integration. However, inactivating the c-NHEJ pathway alone is not sufficient to block T-DNA integration in plants, but the function of Mre11 needs to be inactivated to prevent integration by an alternative integration pathway. The Mre11 protein plays a role in b-NHEJ and likely a role in TMEJ. Our findings offer an alternative way to prevent T-DNA integration, viz. by inactivating or inhibiting both Ku and Mre11. For both components inhibitors have already been developed.

### Example 2: Homologous recombination

Example 1 demonstrates that functional mre11 and Ku80 are required for stable, random integration of T-DNA. The present example demonstrates that functional mre11 and Ku80 are not required for stable integration via homologous recombination.

A T-DNA construct is prepared, containing around 6kb of homology to the endogenous *Arabidopsis* locus protophorphyrinogen oxidase (PPO). The homologous region contains two point mutations, which confer resistance to the herbicide butafenicil upon integration via homologous recombination at the PPO locus (see Hanin et al, Plant J 2001 for a description of the mutations). In addition, the T-DNA encodes a Cas9 enzyme (Arabidopsis codon-optimized Cas9- *AteCas9* (Fauser et al. Plant J 79:348-359 2014)) and guide RNA, directing the Cas9 enzyme to the PPO locus. The expression of Cas9 is driven by the ubiquitin promoter and the guide RNA is under control of the U6 (AtU6) promoter.
Wild type (Col-0) plants and two knock-out alleles of mre11 and two knock-out alleles of Ku80 are transformed by floral dip transformation. Stable integration of the T-DNA via homologous recombination is measured by resistance against butafenicil. The level of stable integration at the site specific locus between wild-type and Ku80-mre11 double mutant plants will be similar.

### Example 3: Homologous recombination

A combination of functional Pol θ and/or Ku80 are required for stable, random integration of T-DNA. The present example demonstrates that functional Pol θ and/or Ku80 are not required for stable integration via homologous recombination.

A T-DNA construct is prepared, containing around 6kb of homology to the endogenous *Arabidopsis* locus protophorphyrinogen oxidase (PPO). The homologous region contains two point mutations, which confer resistance to the herbicide butafenicil upon integration via homologous recombination at the PPO locus (see Hanin et al, Plant J 2001 for a description of the mutations). In addition, the T-DNA encodes a Cas9 enzyme (Arabidopsis codon-optimized Cas9- *AteCas9* (Fauser et al. Plant J 79:348-359 2014)) and guide RNA, directing the Cas9 enzyme to the PPO locus. The expression of Cas9 is driven by the ubiquitin promoter and the guide RNA is under control of the U6 (AtU6) promoter.

Wild type (Col-0) plants and two knock-out alleles of Pol θ (*teb-5* and *teb-2*¹⁷) and/or two knock out alleles of Ku80 are transformed by floral dip transformation. Stable integration of the T-DNA via homologous recombination is measured by resistance against butafenicil.

The level of stable integration at the site specific locus between wild-type and Ku80-Pol θ double mutant plants will be similar.

### Example 4: Transfection with a linearized plasmid

A plasmid is prepared, containing around 6Kb of homology to the endogenous Arabidopsis locus protophorphyrinogen oxidase (PPO). The homologous region contains two point mutations, which confer resistance to the herbicide butafenicil upon integration via homologous recombination at the PPO locus (see Hanin et al, Plant J 2001 for a description of the mutations). In addition, the plasmid encodes a Cas9 enzyme (Arabidopsis codon-optimized Cas9- AteCas9 (Fauser et al. Plant J 79:348-359 2014)) and guide RNA, directing the Cas9 enzyme to the PPO locus. The expression of Cas9 is under control of the ubiquitin promoter and the guide RNA is under control of the U6 (AtU6) promoter. The plasmid also contains a unique restriction site. The plasmid is linearized by digestion with the appropriate restriction site.

Protoplasts are prepared from WT and Ku80-Pol Θ-deficient plants (teb-5 and teb-2) and the linearized plasmid is transformed into the protoplasts by a standard PEG transformation protocol. Colonies are maintained in butafenicil-containing medium to select for stable integration events via homologous recombination. The level of stable integration at the site specific locus between wild-type and Ku80-Pol θ double mutant plants will be similar.

### Example 5: Transfection of tomato plant

The present example will demonstrate the reduction of random integration in a crop plant, namely tomato (Solanum lycopersum). The POLQ gene from tomato was identified from the NCBI database as accession no. XP_010325163 based on a BLAST search using the Tebichi sequence. The *Ku70* gene from tomato was identified form the NCBI database as accession no. XP_004247997 based on a BLAST search. The *Ku80* gene from Tomato was identified form the NCBI database as accession no. XP_004229815 based on a BLAST search.

Ku80-Pol Θ-deficient tomato mutants are created by targeting the Pol Θ locus and the KU80 locus using CRISPR and self-pollinating to create homozygous mutants in the next generation. Briefly, a T-DNA construct is prepared encoding a kanamycin-selectable marker, a Cas9 enzyme (plant codon-optimized Cas9- pcoCas9 (Li et al. 2013 Nat Biotechnol 31:688-691)) and a guide RNA, directing the Cas9 enzyme to the POLQ locus and a guide RNA, directing the Cas9 enzyme to the Ku80 locus. The expression of Cas9 is under control of the 35S promoter and the guide RNAs are under control of the U3 (AtU3) promoter. Tomato cotyledon explants are transformed by immersion in Agrobacterium suspension, selected for kanamycin resistance, and screened for POLQ and Ku80 mutations. Plantlets are screened for POLQ and Ku80 mutations using the Surveyor assay (Voytas 2013 Annu Rev Plant Biol 64:327-350) and plantlets containing an inactivating mutation in POLQ and Ku80 are grown and self-pollinated to create homozygous mutants in the next generation.

The effect of POLQ and Ku80 on random integration is demonstrated using a tumor assay performed on wild-type tomato plants and on Ku80-Pol Θ-deficient tomato mutants. Briefly, Agrobacterium is inserted into a hole in the stem of wild-type tomato plants and Ku80-Pol Θ-deficient tomato plants. Random integration is measured by measuring tumor formation. WT tomato plants will develop tumors-indicating random integration events, while Ku80-Pol Θ-deficient tomato plants will not develop tumors. A similar experiment can be performed targeting the Ku70 locus instead of the Ku80 locus.

### Example 6: Generation of PolQ deficient crop plants

A crop plant, e.g. wheat, soybean, rice, cotton, corn or brassica plant having a mutation in one or more **PolQ** genes (e.g. in one or more homologous genes) is identified or generated via (random) mutagenesis or targeted knockout (e.g. using a sequence specific nuclease such as a meganuclease, a zinc finger nuclease, a TALEN, Crispr/Cas9, Crispr/Cpf1 etc). Reduction in PolQ expression and/or activity is confirmed by Q-PCR, western blotting or the like.

A crop plant, e.g. wheat, soybean, rice, cotton or brassica plant, is transformed with a construct encoding a PolQ inhibitory nucleic acid molecule or PolQ binding molecule (e.g. encoding a PolQ hairpin RNA, antibody, etc, under control of a constitutive or inducible promoter). Reduction in PolQ expression and/or activity is confirmed by Q-PCR, western blotting or the like.

### Example 7: Generation of Ku70 deficient crop plants

A crop plant, e.g. wheat, soybean, rice, cotton, corn or brassica plant having a mutation in one or more Ku70 genes (e.g. in one or more homologous genes) is identified or generated via (random) mutagenesis or targeted knockout (e.g. using a sequence specific nuclease such as a meganuclease, a zinc finger nuclease, a TALEN, Crispr/Cas9, Crispr/Cpf1 etc). Reduction in Ku70 expression and/or activity is confirmed by Q-PCR, western blotting or the like.

A crop plant, e.g. wheat, soybean, rice, cotton or brassica plant, is transformed with a construct encoding a Ku70 inhibitory nucleic acid molecule or Ku70 binding molecule (e.g. encoding a Ku70 hairpin RNA, antibody, etc, under control of a constitutive or inducible promoter). Reduction in Ku70 expression and/or activity is confirmed by Q-PCR, western blotting or the like.

### Example 8: Transfection of polQ deficient crop plants with a selectable marker gene

A plant having reduced PolQ and Ku70 expression (see Example 5), as well as a corresponding plant having wildtype PolQ and Ku 70 expression are transformed with a selectable marker gene (e.g. bar, gus) using Agrobacterium, according to methods well known in the art.

Transformants are screened for expression of the selectable marker gene. Transformants with reduced PolQ and Ku70 expression show transient expression of the selectable marker gene, while transformants with wildtype PolQ and Ku70 expression show stable expression of the selectable marker gene.

Genomic integration of the selectable marker gene is evaluated via e.g. southern blotting of genomic DNA isolated from transformed plants with wildtype or reduced PolQ and Ku70 expression. The presence of the selectable marker gene can be detected in the genomic DNA of PolQ and Ku70-wildtype plants, while plants with reduced PolQ and Ku70 expression show less or even no genomic integration of the selectable marker gene.

### Example 9: Targeted insertion via homologous recombination in PolQ deficient crop plants

PolQ and Ku70 deficient plants (see Example 5) and corresponding plants having wildtype PolQ and Ku70 expression are transformed with a nucleic acid of interest for targeted integration via homologous recombination. The nucleic acid of interest comprises sequences homologous to the genomic DNA at the genomic target site. Optionally, the plant is cotransformed with an expression construct for a sequence specific nuclease capable of inducing a DNA break at the target site to enhance homologous recombination. Plants are screened for integration of the nucleic acid of interest by e.g. southern blotting or PCR. Plants having wild-type PolQ and Ku70 expression show random insertion of nucleic acid of interest as well as targeted insertion via homologous recombination at the target site. Plants with reduced PolQ and Ku70 expression show less or even no random insertion of the nucleic acid of interest, but do show targeted insertion via homologous recombination of the nucleic acid of interest at the target site.

### Example 10: Transfection of polQ and Ku70 deficient crop plants with a selectable marker and the ipt gene

A plant having reduced PolQ and Ku70 expression (see Example 5), as well as a corresponding plant having wildtype PolQ and Ku 70 expression are transformed with a selectable marker (e.g. bar) and the *ipt* gene (determining cytokinin production and thus shoot formation) using Agrobacterium, according to methods well known in the art. Transformants are screened for expression of the selectable marker gene, grown in hormone free medium.

Transformants with wildtype PolQ and Ku70 expression will demonstrate shoot formation and stable expression of the selectable marker gene. Shoots will be abnormal because of the continued expression of the *ipt* gene.

The majority, if not all, of the transformants with reduced PolQ and Ku70 expression will only show transient expression of the selectable marker gene and the *ipt* gene and will thus have a normal phenotype. Genomic integration of the selectable marker and the *ipt* gene is evaluated via e.g. Southern blotting of genomic DNA isolated from transformed plants with wildtype or reduced PolQ and Ku70 expression. The presence of the selectable marker gene will be detected in the genomic DNA of PolQ and Ku70-wildtype plants, while few plants with reduced PolQ expression will have genomic integration of the selectable marker gene and none or very few plants with reduced expression of both PolQ and Ku70 will have the selection marker integrated..

### References

- van Attikum, H., P. Bundock, and P. J. Hooykaas, (2001) Non-homologous end-joining proteins are required for Agrobacterium T-DNA integration. EMBO J. 20: 6550-8.
- van Attikum, H., and Hooykaas, P.J.J. (2003) Genetic requirements for the targeted integration of Agrobacterium T-DNA in Saccharomyces cerevisiae. Nucleic Acids Res., 31, 826-832.Audebert M, Salles B, Calsou P (2004) Involvement of poly(ADP-ribose) polymerase-1 and XRCC1/DNA ligase III in an alternative route for DNA double-strand breaks rejoining. J Biol Chem 279:55117-55126.
- Boulton, S. J., and S. P. Jackson, (1996) Saccharomyces cerevisiae Ku70 potentiates illegitimate DNA doublestrand break repair and serves as a barrier to error-prone DNA repair pathways. EMBO J. 15: 5093-5103.
- Bundock, P., and P. Hooykaas, (2002) Severe developmental defects, hypersensitivity to DNA-damaging agents, and lengthened telomeres in Arabidopsis MRE11 mutants. Plant Cell 14: 2451-2462.
- Ceccaldi, R., B. Rondinelli, and A. D. D'Andrea, (2016) Repair pathway choices and consequences at the double strand break. 338 Trends Cell Biol. 26: 52-64.
- Chamankhah, M., and W. Xiao, (1999) Formation of the yeast Mre11-Rad50-Xrs2 complex is correlated with DNA repair and telomere maintenance. Nucleic Acids Res. 27: 2072-2079.
- Charbonnel, C., M. E. Gallego, and C. I. White, (2010) Xrcc1-dependent and Ku-dependent DNA double-strand break repair kinetics in Arabidopsis plants. Plant J. 64: 280-290.
- Deriano, L., and D. B. Roth, (2013) Modernizing the nonhomologous end-joining repertoire: alternative and classical NHEJ share the stage. Annu. Rev. Genet. 47: 433-55.
- Déry U, Coulombe Y, Rodrigue A, Stasiak A, Richard S, Masson JY., (2008) A glycine-arginine domain in control of the human MRE11 DNA repair protein. Mol Cell Biol. May;28(9):3058-69.
- Gallego, M. E., J.-Y. Bleuyard, S. Daoudal-Cotterell, N. Jallut, and C. I. White, (2003) Ku80 plays a role in non homologous recombination but is not required for T-DNA integration in Arabidopsis. Plant J. 35: 557-565.
- Gallego, M. E., M. Jeanneau, F. Granier, D. Bouchez, N. Bechtold et al., (2001) Disruption of the Arabidopsis RAD50 gene leads to plant sterility and MMS sensitivity. Plant J. 25: 31-41.
- James, P., J. Halladay, and E. A. Craig, (1996) Genomic libraries and a host strain designed for highly efficient two-hybrid selection in yeast. Genetics 144: 1425-1436.
- Jia, Q., P. Bundock, P. J. J. Hooykaas, and S. de Pater, (2012) Agrobacterium tumefaciens T-DNA integration and gene targeting in Arabidopsis thaliana non-homologous end-Joining mutants. J. Bot. 2012: 1-13.
- Jia, Q., A. Den Dulk-Ras, H. Shen, P. J. J. Hooykaas, and S. de Pater, (2013) Poly(ADP-ribose)polymerases are involved in microhomology mediated back-up non-homologous end joining in Arabidopsis thaliana. Plant Mol. Biol. 82: 339-351.
- Mestiri, I., Norre, F., Gallego, M. E. & White, C. I. Multiple host-cell recombination pathways act in Agrobacterium mediated transformation of plant cells. Plant J. (2014). doi:10.1111/tpj.12398
- Nussenzweig A, Nussenzweig MC (2007) A backup DNA repair pathway moves to the forefront. Cell 131:223-225.
- Park, Y. B., J. Chae, Y. C. Kim, and Y. Cho, (2011) Crystal structure of human Mre11: Understanding tumorigenic mutations. Structure 19: 1591-1602.
- Park, S. Y., Z. Vaghchhipawala, B. Vasudevan, L. Y. Lee, Y. Shen et al., (2015) Agrobacterium T-DNA integration into the plant genome can occur without the activity of key non-homologous end-joining proteins. Plant J. 81: 934-946.
- Puizina, J., J. Siroky, P. Mokros, D. Schweizer, and K. Riha, (2004) Mre11 deficiency in Arabidopsis is associated with chromosomal instability in somatic cells and Spo11-dependent genome fragmentation during meiosis. Plant Cell 16: 1968-78.
- 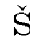 amanić, I., J. Simunić, K. Riha, and J. Puizina, (2013) Evidence for distinct functions of MRE11 in Arabidopsis meiosis. PLoS One 8 (10): e78760 .
- Stracker, T. H., and J. H. J. Petrini, (2011) The MRE11 complex: starting from the ends. Nat. Rev. Mol. Cell Biol. 12: 90-103.
- Tamura K, Adachi Y, Chiba K, Oguchi K, Takahashi H. (2002) Identification of Ku70 and Ku80 homologues in Arabidopsis thaliana: evidence for a role in the repair of DNA double-strand breaks. Plant J. Mar;29(6):771-81.
- Vergunst, A. C., B. Schrammeijer, A. den Dulk-Ras, C. M. T. de Vlaam, T. J. G. Regensburg-Tuihk et al., (2000) VirB/D4-dependent protein translocation from Agrobacterium into plant cells. Sci. 290 : 979-982.
- Waterworth, W. M., C. Altun, S. J. Armstrong, N. Roberts, P. J. Dean et al., (2007) NBS1 is involved in DNA repair and plays a synergistic role with ATM in mediating meiotic homologous recombination in plants. Plant J. 52: 41-52.
- Weijers, D., M. Franke-van Dijk, R. J. Vencken, A. Quint, P. Hooykaas et al., (2001) An Arabidopsis Minute-like phenotype caused by a semi-dominant mutation in a RIBOSOMAL PROTEIN S5 gene. Development 128: 4289-4299.
- Williams, G. J., R. S. Williams, J. S. Williams, G. Moncalian, A. S. Arvai et al., (2011) ABC ATPase signature helices in Rad50 link nucleotide state to Mre11 interface for DNA repair. Nat. Struct. Mol. Biol. 18: 423-31.
- Yousefzadeh MJ, Wood RD. (2013) DNA polymerase POLQ and cellular defense against DNA damage. DNA Repair. Jan 1;12(1):1-9.
- Yu Z, Vogel G, Coulombe Y, Dubeau D, Spehalski E, Hébert J, Ferguson DO, Masson JY, Richard S. (2012) The MRE11 GAR motif regulates DNA double-strand break processing and ATR activation. Cell Res. Feb;22(2):305-20.
- Zha, S., C. Boboila, and F. W. Alt, (2009) Mre11: roles in DNA repair beyond homologous recombination. Nat. Struct. Mol. Biol. 16: 798-800.

## Claims

1. A method for reducing random integration of transfected nucleic acid molecules in a plant cell, said method comprising transfecting a plant cell with a nucleic acid molecule, wherein expression and/or activity in said plant cell is reduced for a component of the classical NHEJ pathway and wherein expression and/or activity in said plant cell is reduced for a component of the POLQ pathway, as compared to a wild-type plant cell, preferably wherein a component of the classical NHEJ pathway is Ku80 and/or Ku70 and wherein a component of the POLQ. pathway is POLQ.

2. A method for transfecting a plant cell with a nucleic acid molecule, the method comprising transfecting a plant cell with a nucleic acid molecule, wherein expression and/or activity for a component of the classical NHEJ pathway is reduced and expression and/or activity of a component of the POLQ pathway is reduced, in said plant cell as compared to a wild-type plant cell.

3. A method for expressing an RNA molecule or a polypeptide in a plant cell, the method comprising transfecting a plant cell with a nucleic acid molecule encoding said RNA molecule or polypeptide, wherein expression and/or activity of a component of the classical NHEJ pathway is reduced and expression and/or activity of a component of the POLQ pathway is reduced, in said plant cell as compared to a wild-type plant cell.

4. A method for producing a plant expressing an RNA molecule or a polypeptide, the method comprising transfecting a plant cell with a nucleic acid molecule encoding said RNA molecule or polypeptide, wherein expression and/or activity of a component of the classical NHEJ pathway is reduced and expression and/or activity of a component of the POLQ pathway is reduced, in said plant cell as compared to a wild-type plant cell, and generating the plant from said plant cell.

5. A method according to any one of the preceding claims, wherein the component of the classical NHEJ pathway is Ku80, Ku70 and/or Lig4.

6. A method according to any one of the preceding claims, wherein the component of the POLQ. pathway is POLQ. and/or mre11.

7. A method according to any one of the preceding claims, wherein said nucleic acid molecule is transiently transfected into the plant cell.

8. A method according to any one of claims 1-6, wherein said nucleic acid molecule is integrated via site-specific genetic recombination or homologous recombination in the chromosome of the plant cell.

9. A method according to any one of the preceding claims, wherein said plant cell comprises an antisense oligonucleotide specific for a pre-mRNA encoded by a gene selected from a component of the classical NHEJ pathway and/or a component of the POLQ pathway, or a double-stranded RNAi molecule specific for mRNA encoded by a component of the classical NHEJ pathway and/or a component of the POLQ pathway.

10. A method according to any one of claims 1-8, wherein the plant cell has one or more mutated alleles for a component of the classical NHEJ pathway and/or a component of the POLQ pathway, such that the expression and/or activity of said genes is reduced as compared to the wild-type genes.

11. A plant produced by the method according to any one of claims 6-10 which comprises said transfected nucleic acid molecule.

12. Plant or plant cell wherein expression and/or activity of a component of the classical NHEJ pathway is reduced and expression and/or activity of a component of the POLQ pathway is reduced, in said plant or plant cell as compared to a wild-type plant cell.

13. A method of producing a plant or plant cell according to claim 12, comprising mutating one or more alleles selected from a component of the classical NHEJ pathway and/or a component of the POLQ pathway in the plant or plant cell or providing the plant or plant cell with an inhibitory nucleic acid molecule or binding molecule for a component of the classical NHEJ pathway and/or a component of the POLQ pathway.

14. Use of the plant or plant cell according to claim 12 or produced according to claim 13 for producing a plant expressing an RNA molecule or a polypeptide, wherein the nucleic acid molecule encoding said RNA molecule or polypeptide is not integrated into the plant cell chromosome.

15. Use of the plant or plant cell according to claim 12 or produced according to claim 13 for producing a plant expressing an RNA molecule or a polypeptide, wherein the nucleic acid molecule encoding said RNA molecule or polypeptide is integrated via site-specific genetic recombination or homologous recombination in the chromosome of the plant cell.
